# EUROPEAN PATENT SPECIFICATION

(11) **EP 3 243 905 B1**
(45) Date of publication and mention of the grant of the patent: **16.11.2022**
(21) Application number: 17176405.3
(22) Date of filing: 03.04.2008
(51) Int. Cl.: C12N 15/82, C12N 9/10, C12N 9/88, A01H 5/00, A01H 5/10

(54) **HERBICIDE-RESISTANT BRASSICA PLANTS AND METHODS OF USE**
HERBIZIDRESISTENTE BRASSICA-PFLANZEN UND VERFAHREN ZU DEREN VERWENDUNG
PLANTS DE BRASSICA RÉSISTANTS AUX HERBICIDES ET PROCÉDÉS D'UTILISATION

(30) Priority: 04.04.2007 US 910008 P
(43) Date of publication of application: 15.11.2017
(62) Divisional of application: 12165378.6
(73) Proprietor: BASF SE, 67056 Ludwigshafen am Rhein (DE); Viterra, Inc., Regina, SK S4T 5T9 (CA)
(72) Inventor: Yao, Kening, Saskatoon, Saskatchewan S7N 4H8 (CA); Potts, Derek, Saskatoon, Saskatchewan S7H 0P3 (CA); Males, Daryl, Saskatoon, Saskatchewan S7M 3R4 (CA)
(74) Representative: Altmann Stößel Dick Patentanwälte PartG mbB

(56) References cited:
- WO-A2-2007/005581
- US-A1- 2003 097 692
- US-A1- 2005 283 858
- WIERSMA P A ET AL: "ISOLATION, EXPRESSION AND PHYLOGENETIC INHERITANCE OF AN ACETOLACTATE SYNTHASE GENE FROM BRASSICA NAPUS", MOLECULAR AND GENERAL GENETICS, SPRINGER VERLAG, BERLIN, DE, vol. 219, no. 3, 1 November 1989 (1989-11-01), pages 413-420, XP000095145, ISSN: 0026-8925, DOI: 10.1007/BF00259614
- Alan K. Chang ET AL: "Herbicide-resistant forms of Arabidopsis thaliana acetohydroxyacid synthase : characterization of the catalytic properties and sensitivity to inhibitors of four defined mutants", , 1 August 1998 (1998-08-01), pages 765-777, XP055010376, Retrieved from the Internet: URL:http://www.biochemj.org/bj/333/0765/33 30765.pdf [retrieved on 2011-10-25]
- CHONG C-K ET AL: "Amino acid residues conferring herbicide tolerance in tobacco acetolactate synthase", BIOCHEMICAL AND BIOPHYSICAL RESEARCH COMMUNICATIONS, ACADEMIC PRESS INC. ORLANDO, FL, US, vol. 279, no. 2, 20 December 2000 (2000-12-20), pages 462-467, XP002224077, ISSN: 0006-291X, DOI: 10.1006/BBRC.2000.3958
- TAN S ET AL: "IMIDAZOLINONE-TOLERANT CROPS: HISTORY, CURRENT STATUS AND FUTURE", PEST MANAGEMENT SCIENCE, WILEY & SONS, BOGNOR REGIS, GB, vol. 61, no. 3, 1 January 2005 (2005-01-01), pages 246-257, XP009058795, ISSN: 1526-498X

## Description

### CROSS-REFERENCE TO RELATED APPLICATIONS

This application claims the benefit of U.S. Provisional Application No. 60/910,008, filed April 4, 2007.

### FIELD OF THE INVENTION

This invention relates to herbicide-resistant *Brassica juncea* plants, seeds, and methods using such plants.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (Singh (1999) "Biosynthesis of valine, leucine and isoleucine," in Plant Amino Acid, Singh, B.K., ed., Marcel Dekker Inc. New York, New York, pp. 227-247). AHAS is the site of action of five structurally diverse herbicide families including the sulfonylureas (Tan et al. (2005) Pest Manag. Sci. 61:246-57; Mallory-Smith and Retzinger (2003) Weed Technology 17:620-626; 'LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288), Tan et al. (2005) Pest Manag. Sci. 61:246-57; Mallory-Smith and Retzinger (2003) Weed Technology 17:620-626, the sulfonylamino-carbonyltriazolinones (Tan et al. (2005) Pest Manag. Sci. 61:246-57; Mallory-Smith and Retzinger (2003) Weed Technology 17:620-626). Imidazolinone and sulfonylurean herbicides are widely used in modern agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT^{®} (imazethapyr), SCEPTER^{®} (imazaquin) and ARSENAL^{®} (imazapyr). Examples of sulfonylurean herbicides are chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl and halosulfuron.

Due to their high effectiveness and low-toxicity, imidazolinone herbicides are favored for application by spraying over the top of a wide area of vegetation. The ability to spray an herbicide over the top of a wide range of vegetation decreases the costs associated with plantation establishment and maintenance, and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone-resistant species of the desired vegetation in the spray over area.

Among the major agricultural crops, some leguminous species such as soybean are naturally resistant to imidazolinone herbicides due to their ability to rapidly metabolize the herbicide compounds (Shaner and Robinson (1985) Weed Sci. 33:469-471). Other crops such as corn (Newhouse et al. (1992) Plant Physiol. 100:882886) and rice (Barrett et al. (1989) Crop Safeners for Herbicides, Academic Press, New York, pp. 195-220) are somewhat susceptible to imidazolinone herbicides. The differential sensitivity to the imidazolinone herbicides is dependent on the chemical nature of the particular herbicide and differential metabolism of the compound from a toxic to a non-toxic form in each plant (Shaner et al. (1984) Plant Physiol. 76:545-546; Brown et al., (1987) Pestic. Biochem. Physiol. 27:24-29). Other plant physiological differences such as absorption and translocation also play an important role in sensitivity (Shaner and Robinson (1985) Weed Sci. 33:469-471).

Plants resistant to imidazolinones, sulfonylureas and triazolopyrimidines have been successfully produced using seed, microspore, pollen, and callus mutagenesis in *Zea mays, Arabidopsis thaliana, Brassica napus (i.e.,* canola) *Glycine max, Nicotiana tabacum,* and *Oryza sativa* (Sebastian et al. (1989) Crop Sci. 29:1403-1408; Swanson et al., 1989 Theor. Appl. Genet. 78:525-530; Newhouse et al. (1991) Theor. Appl. Genet. 83:65-70; Sathasivan et al. (1991) Plant Physiol. 97:1044-1050; Mourand et al. (1993) J Heredity 84:91-96; U.S. Patent No. 5,545,822). In all cases, a single, partially dominant nuclear gene conferred resistance. Four imidazolinone resistant wheat plants were also previously isolated following seed mutagenesis of *Triticum aestivum* L. cv. Fidel (Newhouse et al. (1992) Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al. (1992) Plant Physiol. 100:882-886).

Computer-based modeling of the three dimensional conformation of the AHAS-inhibitor complex predicts several amino acids in the proposed inhibitor binding pocket as sites where induced mutations would likely confer selective resistance to imidazolinones (Ott et al. (1996) J. Mol. Biol. 263:359-368). Wheat plants produced with some of these rationally designed mutations in the proposed binding sites of the AHAS enzyme have in fact exhibited specific resistance to a single class of herbicides (Ott et al. (1996) J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439 and 6,222,100 generally describe the use of an altered AHAS gene to elicit herbicide resistance in plants, and specifically discloses certain imidazolinone resistant corn lines. U.S. Patent No. 5,013,659 discloses plants exhibiting herbicide resistance due to mutations in at least one amino acid in one or more conserved regions. The mutations described therein encode either cross-resistance for imidazolinones and sulfonylureas or sulfonylurea-specific resistance, but imidazolinone-specific resistance is not described. U.S. Patent No. 5,731,180 and U.S. Patent No. 5,767,361 discuss an isolated gene having a single amino acid substitution in a wild-type monocot AHAS amino acid sequence that results in imidazolinone-specific resistance. In addition, rice plants that are resistant to herbicides that interfere with AHAS have been developed by mutation breeding and also by the selection of herbicide resistant plants from a pool of rice plants produced by anther culture. *See,* U.S. Patent Nos. 5,545,822, 5,736,629, 5,773,703, 5,773,704, 5,952,553 and 6,274,796.

In plants, as in all other organisms examined, the AHAS enzyme is comprised of two subunits: a large subunit (catalytic role) and a small subunit (regulatory role) (Duggleby and Pang (2000) J. Biochem. Mol. Biol. 33:1-36). The AHAS large subunit (also referred to herein as AHASL) may be encoded by a single gene as in the case of *Arabidopsis* and rice or by multiple gene family members as in maize, canola, and cotton. Specific, single-nucleotide substitutions in the large subunit confer upon the enzyme a degree of insensitivity to one or more classes of herbicides (Chang and Duggleby (1998) Biochem J. 333:765-777).

For example, bread wheat, *Triticum aestivum* L., contains three homologous acetohydroxyacid synthase large subunit genes. Each of the genes exhibits significant expression based on herbicide response and biochemical data from mutants in each of the three genes (Ascenzi et al. (2003) International Society of Plant Molecular Biologists Congress, Barcelona, Spain, Ref. No. S10-17). The coding sequences of all three genes share extensive homology at the nucleotide level (WO 03/014357). Through sequencing the *AHASL* genes from several varieties of *Triticum aestivum,* the molecular basis of herbicide tolerance in most IMI-tolerant (imidazolinone-tolerant) lines was found to be the mutation Ser653(*At*)Asn, indicating a serine to asparagine substitution at a position equivalent to the serine at amino acid 653 in *Arabidopsis thaliana* (WO 03/014357). This mutation is due to a single nucleotide polymorphism (SNP) in the DNA sequence encoding the AHASL protein.

Multiple *AHASL* genes are also know to occur in dicotyledonous plant species. Recently, Kolkman et al. ((2004) Theor. Appl. Genet. 109: 1147-1159) reported the identification, cloning, and sequencing for three *AHASL* genes *(AHASL 1, AHASL2,* and *AHASL3)* from herbicide-resistant and wild type genotypes of sunflower *(Helianthus annuus* L.). Kolkman *et al.* reported that the herbicide-resistance was due either to the Pro 197Leu (using the *Arabidopsis* AHASL amino acid position nomenclature) substitution or the Ala205Val substitution in the AHASLI protein and that each of these substitutions provided resistance to both imidazolinone and sulfonylurean herbicides. US2003/097692 describes Arabidopsis thaliana genes encoding a mutant acetolactate synthase (ALS) enzyme that is specifically resistant to imidazolinone herbicides. Wiermsa et al. (Molecular and General Genetics 1989, vol 219, p.413-420) describe the isolation, expression and phylogenetic inheritance of an acetolactate synthase gene from Brassica napus. Chang et al. (Biochem J (1998) 333 (3): 765-777) describe herbicide-resistant forms of Arabidopsis thaliana acetohydroxyacid synthase as well as the characterization of the catalytic properties and sensitivity to inhibitors of four defined mutants. Chong and Choi (Biochemical and Biophysical Research Communications, Volume 279, Issue 2, 20 December 2000, Pages 462-467) describe amino acid residues conferring herbicide tolerance in Tobacco acetolactate synthase. WO 2007/005581 describes herbicide-resistant sunflower plants, isolated polynucleotides that encode herbicide-resistant and wild-type acetohydroxyacid synthase large subunit (AHASL) polypeptides, and the amino acid sequences of these polypeptides. Tan et al. (https://doi.org/10.1002/ps.993) describe the history, current status and future of imidazolinone-tolerant crops. US2005/283858 describes Brassica juncea plants, plant parts and plant seeds that are resistant to imidazolinone herbicides. Two B. juncea AHAS gene sequences (BjAHAS-a and BjAHAS-b) and one B. nigra AHAS gene sequence (BngrAHAS) are described. The sequence of the mutant allele, BjAHAS-bR, is also described.

Given their high effectiveness and low-toxicity, imidazolinone herbicides are favored for agricultural use. However, the ability to use imidazolinone herbicides in a particular crop production system depends upon the availability of imidazolinone-resistant varieties of the crop plant of interest. To enable farmers greater flexibility in the types and rates of imidazolinone and sulfonylurean herbicides they use, a stronger herbicide tolerance is often desired. Also, plant breeders who develop herbicide tolerant varieties want to work with mutations that provide greater herbicide tolerance, allowing them greater flexibility in the germplasm backgrounds they use to develop their varieties. To produce such imidazolinone-resistant varieties, plant breeders need to develop additional breeding lines, preferably with increased imidazolinone-resistance. Thus, additional imidazolinone-resistant breeding lines and varieties of crop plants, as well as methods and compositions for the production and use of imidazolinone-resistant breeding lines and varieties, are needed.

### SUMMARYOFTHE INVENTION

The present invention relates to a *Brassica juncea* plant comprising an acetohydroxyacid synthase large subunit (AHASL) polynucleotide encoding a herbicide-tolerant AHASL polypeptide having a threonine substitution at a position corresponding to position 122 of SEQ ID NO: 1, position 107 of SEQ ID NO: 4, or position 104 of SEQ ID NO: 5, wherein the AHASL polynucleotide is non-transgenic, wherein said plant comprises
(a) both (1) an A genome comprising the AHASL polynucleotide encoding the AHASL polypeptide having a threonine substitution at the position corresponding to position 104 of SEQ ID NO: 5 and (2) a B genome comprising an AHASL polynucleotide encoding a herbicide-tolerant AHASL polypeptide having an asparagine substitution at a position corresponding to position 638 of SEQ ID NO: 2, wherein said plant is homozygous for both of said AHASL polynucleotides, or
(b) both (1) a B genome comprising the AHASL polynucleotide encoding the AHASL polypeptide having a threonine substitution at the position corresponding to position 107 of SEQ ID NO: 4 and (2) an A genome comprising an AHASL polynucleotide encoding a herbicide-tolerant AHASL polypeptide having a leucine substitution at a position corresponding to position 556 of SEQ ID NO: 6, wherein said plant is homozygous for both of said AHASL polynucleotides.
is defined by the claims and any information that does not fall within the claims is provided for information only.

In a preferred embodiment, the *Brassica juncea* plant of the invention
a) is a progeny of line J04E-0122, a representative sample of seed of the line having been deposited under ATCC Patent Deposit Number PTA-7944;
b) is a mutant, recombinant, or a genetically engineered derivative of line J04E-0122, or
c) is a progeny of at least one of the plants of a) or b).

In another preferred embodiment, the *Brassica juncea* plant of the invention
a) is a progeny of line J04E-0130, a representative sample of seed of the line having been deposited under ATCC Patent Deposit Number PTA-7945;
b) is a mutant, recombinant, or a genetically engineered derivative of line J04E-0130, or
c) is a progeny of at least one of the plants of a) or b).

The present invention further relates to a plant cell or a plant part of the *Brassica juncea* plant of the invention.

In addition, the invention relates to a seed of the *Brassica juncea* plant of the invention, wherein the seed comprises the AHASL polynucleotides of the *Brassica juncea* plant of the invention.

In a preferred embodiment, said seed is treated with a seed treatment formulation.

Preferably, the seed treatment formulation comprises an herbicide selected from imidazolinones, sulfonylureas, triazolopyrimidines, pyrimidinyloxybenzoates, and mixtures thereof.

The invention further provides a method for growing a *Brassica juncea* plant of the invention, comprising applying an effective amount of an AHAS-inhibiting herbicide to the plant and the vicinity of the plant.

In a preferred embodiment of the method for growing a *Brassica juncea* plant of the invention, the AHAS-inhibiting herbicide comprises one or more of an imidazolinone herbicide, a sulfonylurea herbicide, a triazolopyrimidine herbicide, and a pyrimidinyloxybenzoate herbicide.

### BRIEF DESCRIPTION THE DRAWINGS

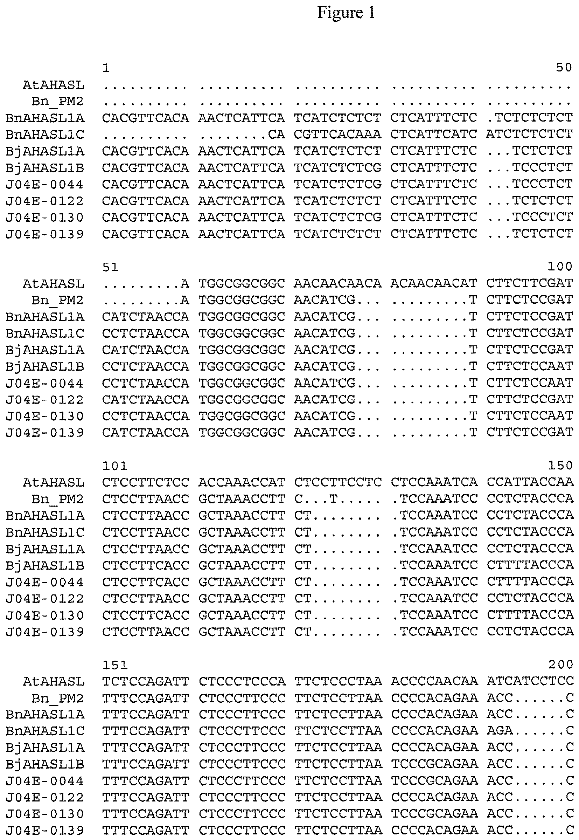

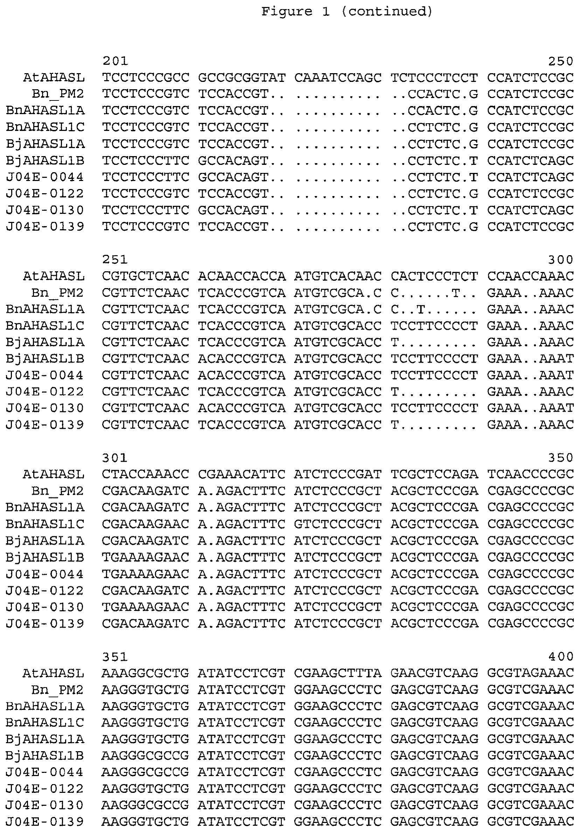

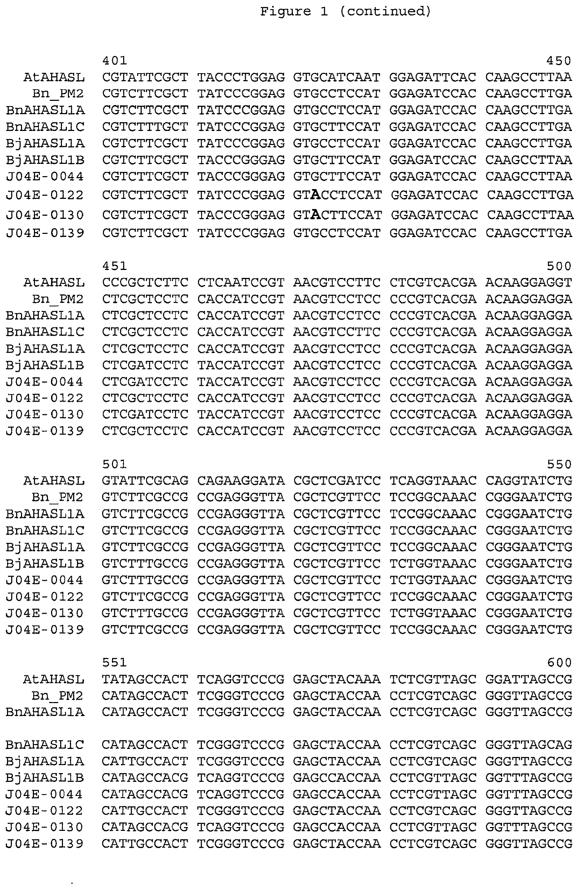

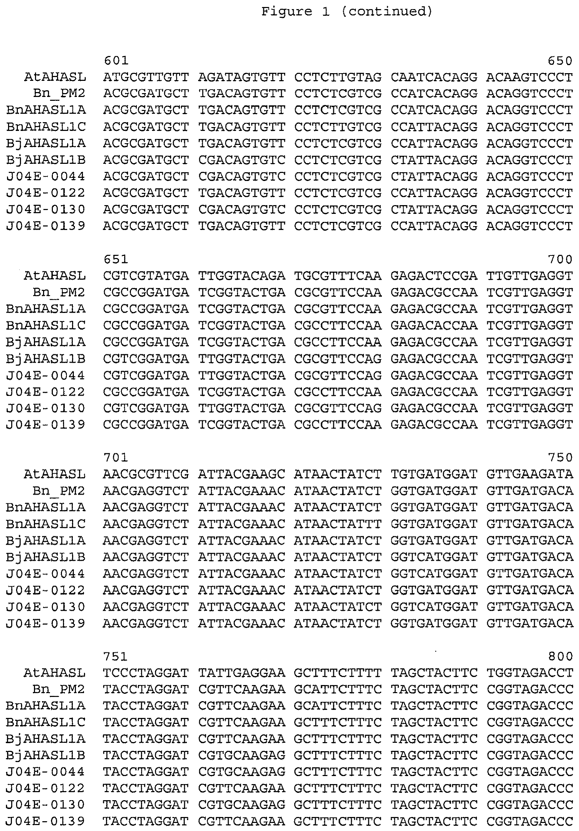

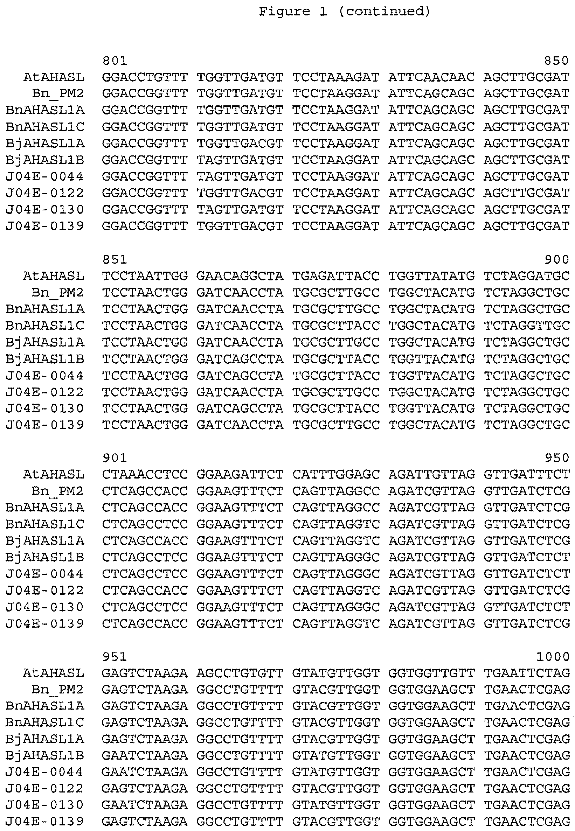

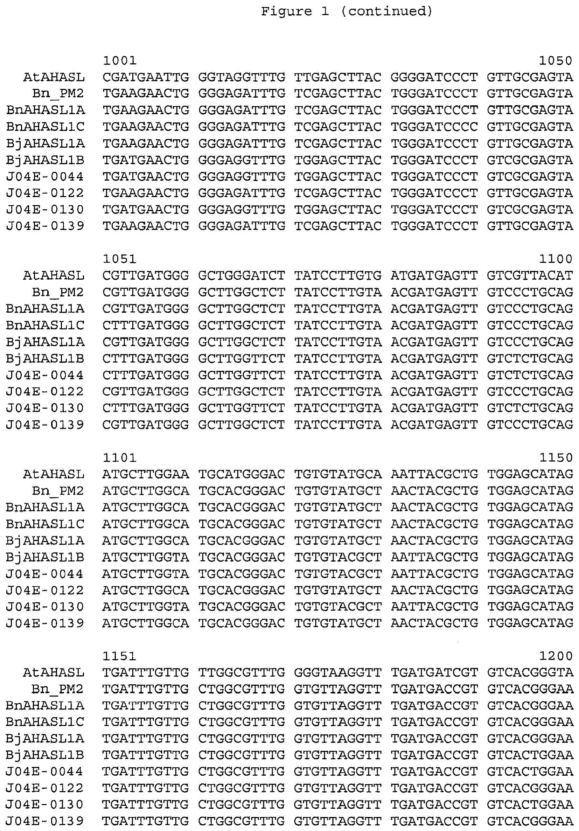

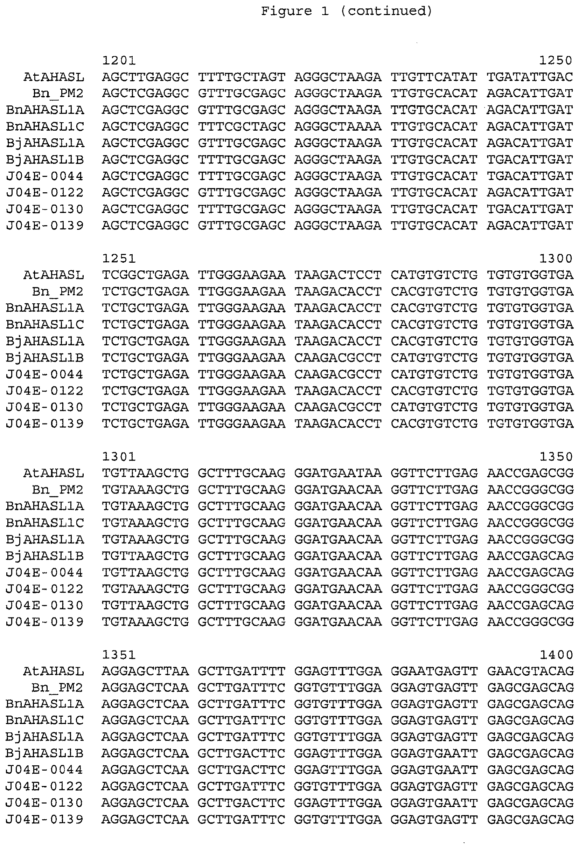

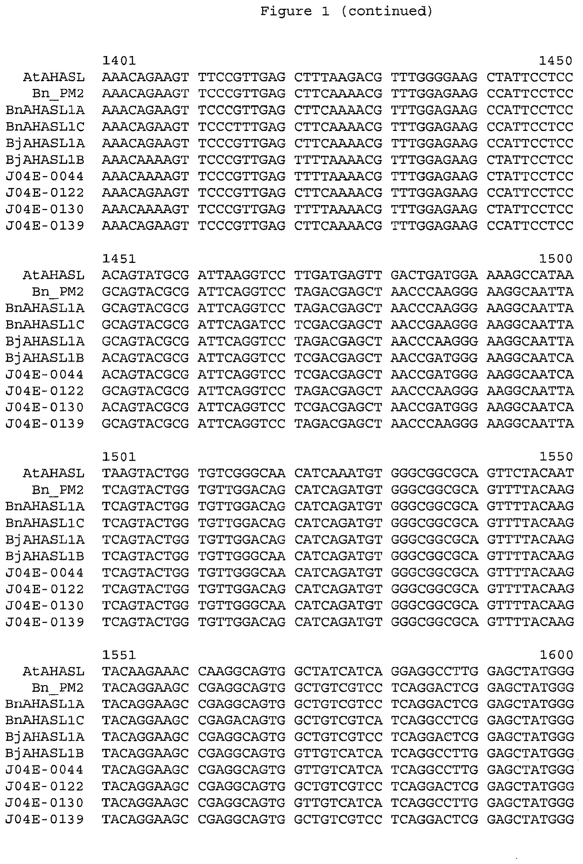

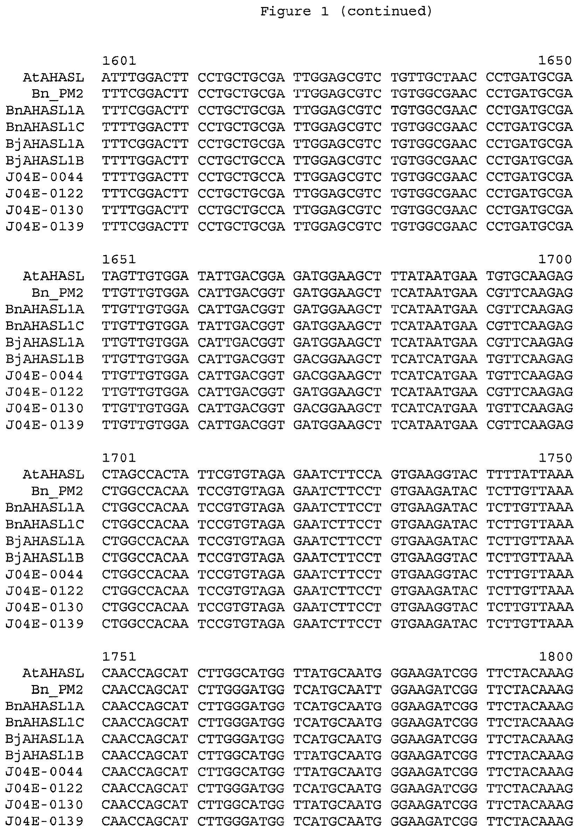

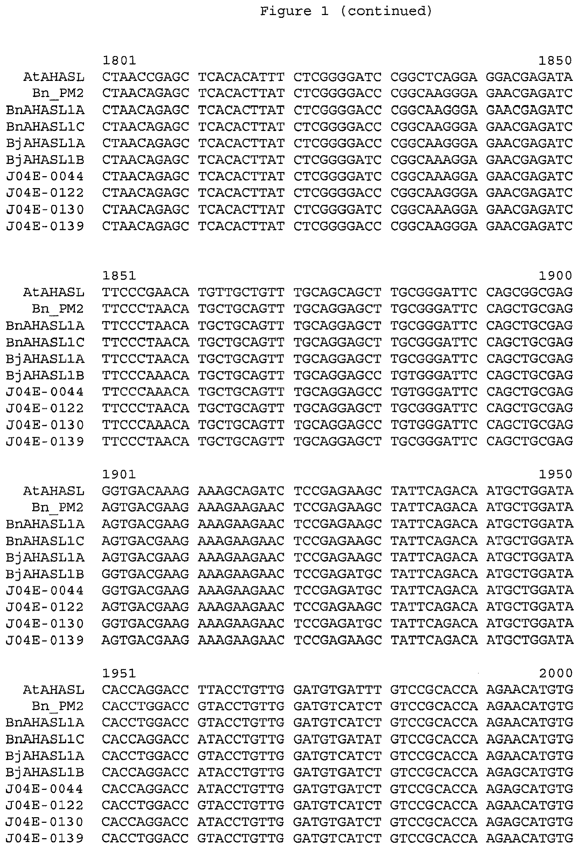

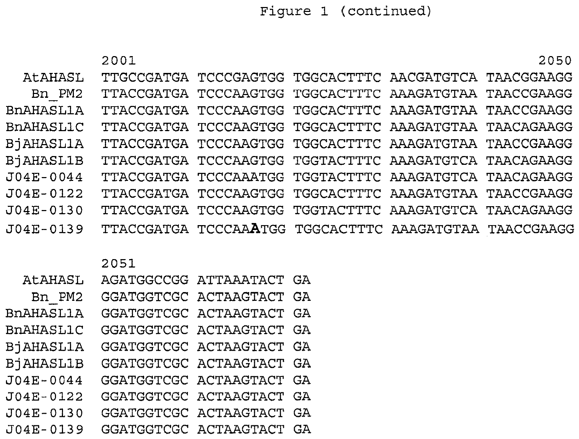

Figure 1 displays an alignment of the nucleotide sequences of the coding regions of the wild-type AHASL gene from *Arabidopsis thaliana* (AtAHASL, SEQ ID NO:11), herbicide-resistant BjAHASL1B-S653N gene of *Brassicajuncea* from line J04E-0044 (J04E-0044, SEQ ID NO:12), herbicide-resistant BjAHASL1A-S653N gene of *Brassica juncea from* line J04E-0139 (J04E-0139, SEQ ID NO:13), herbicide-resistant BjAHASL1B-A122T gene of *Brassica juncea from* line J04E-0130 (J04E-0130, SEQ ID NO:14), herbicide-resistant BjAHASL1A-A122T gene of *Brasscijuncea* from line J04E-0122 (BjAHASLIA, SEQ ID NO:15), herbicide-resistant BnAHASL1A-W574L gene of *Brassica napus* from PM2 line (BnAHASLIA, SEQ ID NO:16), wild-type BjAHASLIA gene of *Brassicajuncea(BjAHASLIA,* SEQ ID NO:17), wild-type BjAHASLIB gene of *Brassicajuncea(BjAHASLIB,* SEQ ID NO:18), wild-type BnAHASLIA gene of *Brassica napus* (BnAHASLIA, SEQ ID NO: 19), wild-type BnAHASLIC gene of *Brassica napus* (BnAHASLIC, SEQ ID NO:20). The analysis was performed in Vector NTI software suite using the Fast Algorithm (gap opening 15, gap extension 6.66 and gap separation 8, matrix is swgapdnamt).

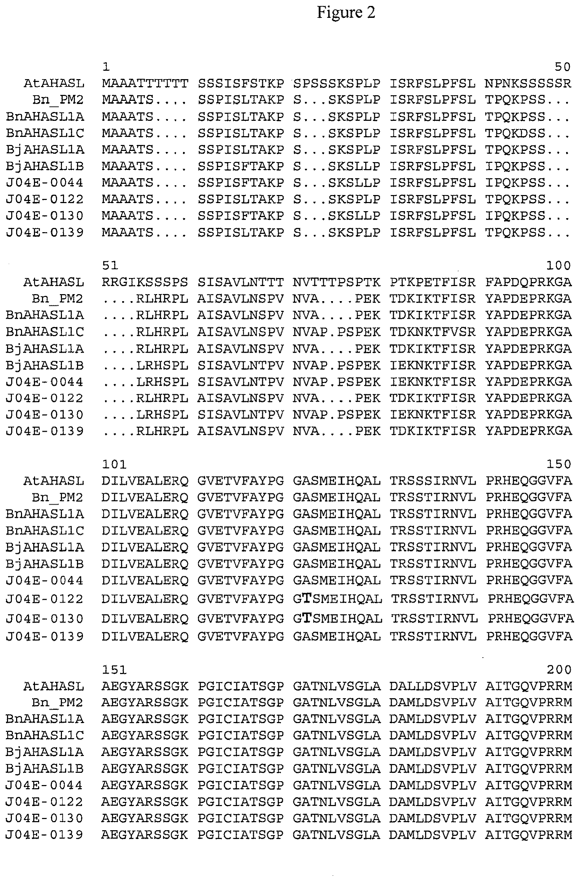

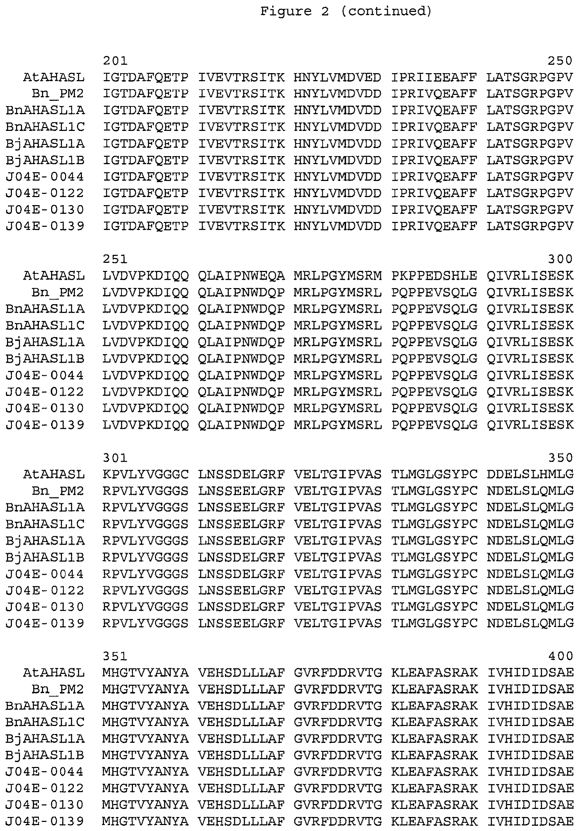

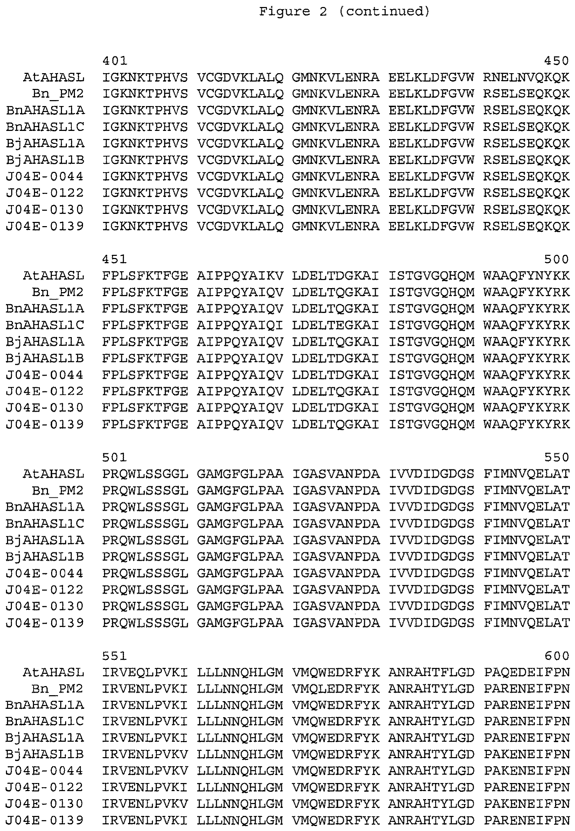

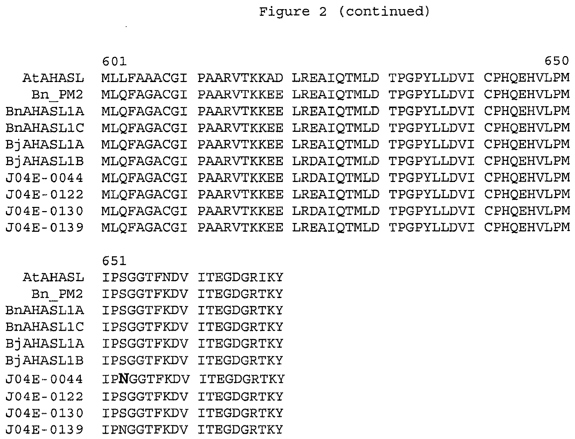

Figure 2 displays an alignment of the amino acid sequences of the wild-type AHASL gene from *Arabidopsis thaliana* (AtAHASL, SEQ ID NO: 1), herbicide-resistant BjAHASL1B-S653N gene of *Brassica juncea from* line J04E-0044 (J04E-0044, SEQ ID NO:2), herbicide-resistant BjAHASL1A-S653N gene of *Brassicajuncea* from line J04E-0139 (J04E-0139, SEQ ID NO:3), herbicide-resistant BjAHASL1B-A122T gene of *Brassica juncea* from line J04E-0130 (J04E-0130, SEQ ID NO:4), herbicide-resistant BjAHASL1A-A122T gene of *Brassci juncea from* line J04E-0122 (J04E-0122, SEQ ID NO:5), herbicide-resistant BnAHASL1A-W574L gene of *Brassica napus* from PM2 line (BnAHASLIA, SEQ ID NO:6), wild-type BjAHASLIA gene of *Brassicajuncea(BjAHASLIA,* SEQ ID NO:7), wild-type BjAHASLIB gene of *Brassicajuncea(BjAHASLIB,* SEQ ID NO:8), wild-type BnAHASLIA gene of *Brassica napus* (BnAHASLI, SEQ ID NO:9), wild-type BnAHASLIC gene of *Brassica napus* (BnAHASLIC, SEQ ID NO:10). The analysis was performed in Vector NTI software suite (gap opening penalty = 10, gap extension penalty = 0.05, gap separation penalty = 8, blosum 62MT2 matrix).
Figure 3 is a bar chart showing the AHAS enzyme activity assay results for *B.juncea* plant lines.
Figure 4 is a chart showing the greenhouse spray assay results for *B.juncea* plant lines.

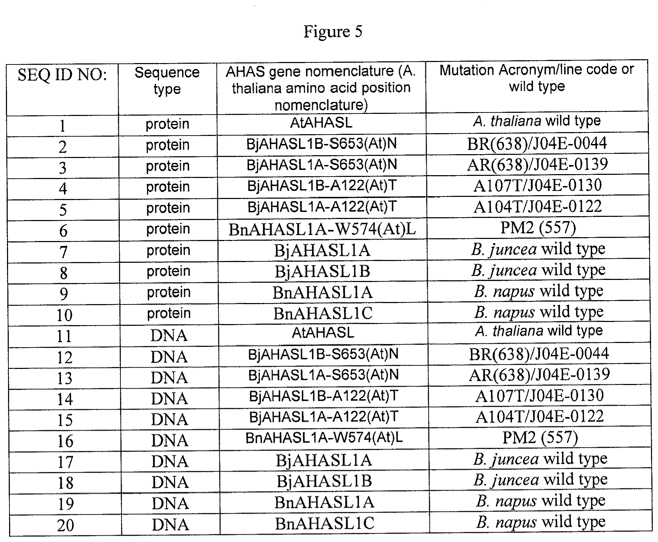

Figure 5 is a table showing the SEQ ID NO to the corresponding DNA or protein sequence.
Figure 6 provides AHAS enzyme activity in protein extracts isolated from homozygous *B.juncea* lines containing combinations of aR, bR, AI07T, and A104T *B.juncea* mutations stacked with each other and with the introgressed PM2 mutation in *B.juncea at* 100 µM of Imazamox.
Figure 7 provides the mean plant injury (Phytotoxcity) of *B.juncea* F2 lines containing different zygosities and combinations of the aR and A107T AHAS mutations 2 weeks post-spray in the greenhouse with 35 g ai/ha of Imazamox.
Figure 8 provides mean plant phytotoxocity of homozygous *B.juncea DH* lines containing combinations of aR, bR, AI07T, and A104T *B.juncea* mutations stacked with each other and with the introgressed PM2 mutation in *B.juncea two* weeks after being sprayed with 35 g ai/ha equivalent Imazamox (Raptor^{®}).

### DETAILED DESCRIPTION OF THE INVENTION

The present invention relates to *Brassica juncea* plants having increased resistance to herbicides when compared to a wild-type *Brassica juncea* plant. Herbicide-resistant *Brassica* plants were produced as described in detail below by exposing isolated, wild-type (with respect to herbicide resistance) *Brassica* microspores to a mutagen, culturing the microspores in the presence of an effective amount of an imidazolinone herbicide, and selecting the surviving embryos. From the surviving embryos, haploid *Brassica* plants were produced and then chromosome doubled to yield fertile, doubled haploid *Brassica* plants that display enhanced resistance to an imidazolinone herbicide, relative to the resistance of a wild-type *Brassica* plant. In one embodiment, the present invention provides an herbicide resistant *Brassica* line referred to herein as J04E-0130 that was produced from the mutagenesis of microspores. In another embodiment, the present invention provides an herbicide resistant *Brassica* line referred to herein as J04E-0122 that was produced from the mutagenesis of microspores.

Thus, the present invention provides *Brassica juncea* plants having resistance to AHAS inhibiting herbicides. *B.juncea* lines are provided that contain mutations in at least one AHASL polynucleotide, in which one mutation is a G-to-A transversion that corresponds to an amino acid at position 653 of the *Arabidposis thaliana* AHASLI sequence and a G-to-A transversion that corresponds to an amino acid at position 122 of the *A. thaliana* AHASLI sequence.

From both J04E-0130 herbicide-resistant *Brassica juncea* plants and wild-type *Brassica juncea* plants, the coding region of an acetohydroxyacid synthase large subunit gene (designated as AHASLI) was isolated by polymerase chain reaction (PCR) amplification and sequenced. By comparing the polynucleotide sequences of the herbicide resistant and wild-type *Brassica* plants, it was discovered that the coding region of the AHASLI polynucleotide sequence from the herbicide resistant *Brassica* plant line J04E-0130 is located on the B genome of *Brassicajuncea* and differs from the AHASLI polynucleotide sequence of the wild type plant by a single nucleotide, a G-to-A transversion (Figure 1). This G-to-A transversion in the AHASLI polynucleotide sequence results in a novel Ala-to-Thr substitution at amino acid 107 (corresponding to amino acid 122 of the *A. thaliana* AHASLI) in a conserved region of the predicted amino acid sequence of the AHASLI protein, relative to the amino acid sequence of the wild-type AHASLI protein (Figure 2).

From both J04E-0122 herbicide-resistant *Brassica juncea* plants and wild-type *Brassica juncea* plants, the coding region of an acetohydroxyacid synthase large subunit gene (designated as AHASLI) was isolated by polymerase chain reaction (PCR) amplification and sequenced. By comparing the polynucleotide sequences of the herbicide resistant and wild-type *Brassica* plants, it was discovered that the coding region of the AHASLI polynucleotide sequence from the herbicide resistant *Brassica* plant line J04E-0122 is located on the A genome of *Brassica juncea and* differs from the AHASLI polynucleotide sequence of the wild type plant by a single nucleotide, a G-to-A transversion (Figure 1). This G-to-A transversion in the AHASLI polynucleotide sequence results in a novel Ala-to-Thr substitution at amino acid 104 (corresponding to amino acid 122 of the *A. thaliana* AHASL 1) in a conserved region of the predicted amino acid sequence of the AHASLI protein, relative to the amino acid sequence of the wild-type AHASLI protein (Figure 2).

The present disclosure also provides *B. juncea* plants that contain at least two mutated AHASL polynucleotides. Such plants are also referred to herein as plants containing "stacked" mutations. The mutations may be on different genomes of the *B.juncea* plant. The *B. juncea* plants may contain any number of mutated AHASL polynucleotides and any combination of mutations, including, but not limited to mutations corresponding to position 653 of SEQ ID NO:1, position 638 of SEQ ID NO:2, position 635 of SEQ ID NO:3, positions 122 of SEQ ID NO:1, position 107 of SEQ ID NO:4, position 104 of SEQ ID NO:5, position 574 of SEQ ID NO:1, or position 556 of SEQ ID NO:6.

Also provided herein are *B.juncea* plants having two mutated AHASL polynucleotides on different genomes, one mutated AHASL polynucleotide on the A genome and the second mutated AHASL polynucleotide on the B. genome.

To overcome the issues associated with transferring an herbicide tolerance trait from the C genome of *Brassica napus* onto the A or B genomes of *Brassica rapa* and/or *Brassica juncea,* it is advantageous to directly produce the mutation in the desired genome. U.S. patent application 2005/0283858 discloses an herbicide tolerant *Brassicajuncea AHAS1* mutation, bR, which was produced by direct mutagenesis resulting in a SNP on the AHASI gene causing a substitution of Ser638Asn (position 653 using the *Arabidopsis* AHASL amino acid position nomenclature) in the AHASL gene on the B genome.

The *B.juncea* plants having two or more AHASL mutations provided herein may have increased levels of herbicide resistance compared to the additive levels of resistance of the individual mutations. Plants having two or more AHASL mutations may have levels of resistance that is 10%, 20%, 25%, 30%, 35%, 40%, 45%, 50%, or more higher compared to the additive levels of resistance provided by the individual AHASL mutations.

The increases in resistance may be measured using any method for determining AHAS resistance. For example, resistance may be measured by determining the percent resistance in *B.juncea* at a time period that is 10, 12, 14, 16, 18, 20, 22, 24, 26, or 28 days or more after treatment with an AHAS inhibiting herbicide. The percent resistance may then be compared to the levels obtained by adding the percent resistance in plants containing the respective individual AHASL mutations. In one aspect, the resistance is determined by measuring the percent resistance in plants 14 days after treatment with a 2x amount of an AHAS-inhibiting herbicide.

The disclosure further relates to isolated polynucleotide molecules comprising nucleotide sequences that encode acetohydroxyacid synthase large subunit (AHASL) proteins and to such AHASL proteins. Disclosed are the isolation and nucleotide sequence of a polynucleotide encoding an herbicide-resistant *Brassica* AHASLI protein from an herbicide-resistant *Brassica* plant that was produced by chemical mutagenesis of wild-type *Brassica* plants. The herbicide-resistant AHASLI proteins of the disclosure possess a serine-to-asparagine substitution at position 635 of the *B.juncea* AHASLI gene located on the A genome, or an alanine-to-threonine substitution at position 107 of the *B.juncea* AHASLI gene located on the B genome, or an alanine-to-threonine substitution at position 104 of the *B.juncea* AHASLI gene located on the A genome. The disclosure further provides the isolation and nucleotide sequence of a polynucleotide molecule encoding a wild-type *Brassica* AHASLI protein.

The present disclosure provides isolated polynucleotide molecules that encode AHASLI proteins from *Brassica,* particularly *Brassica juncea.* Specifically, the disclosure provides isolated polynucleotide molecules comprising: the nucleotide sequence as set forth in SEQ ID NO:13, nucleotide sequences encoding the AHASLI protein comprising the amino acid sequence as set forth in SEQ ID NO: 3, the nucleotide sequence as set forth in SEQ ID NO: 14, nucleotide sequences encoding the AHASLI protein comprising the amino acid sequence as set forth in SEQ ID NO:4, the nucleotide sequence as set forth in SEQ ID NO:15, nucleotide sequences encoding the AHASL1 protein comprising the amino acid sequence as set forth in SEQ ID NO: 5, and fragments and variants of such nucleotide sequences that encode functional AHASLI proteins.

The isolated herbicide-resistant AHASLI polynucleotide molecules of the disclosure comprise nucleotide sequences that encode herbicide-resistant AHASLI proteins. Such polynucleotide molecules can be used in polynucleotide constructs for the transformation of plants, particularly crop plants, to enhance the resistance of the plants to herbicides, particularly herbicides that are known to inhibit AHAS activity, more particularly imidazolinone herbicides. Such polynucleotide constructs can be used in expression cassettes, expression vectors, transformation vectors, plasmids and the like. The transgenic plants obtained following transformation with such polynucleotide constructs show increased resistance to AHAS-inhibiting herbicides such as, for example, imidazolinone and sulfonylurean herbicides.

Compositions of the disclosure include nucleotide sequences that encode AHASLI proteins. In particular, the present disclosure provides for isolated polynucleotide molecules comprising nucleotide sequences encoding the amino acid sequence shown in SEQ ID NO:3, 4, or 5, and fragments and variants thereof that encode polypeptides comprising AHAS activity. Further provided are polypeptides having an amino acid sequence encoded by a polynucleotide molecule described herein, for example the nucleotide sequence set forth in SEQ ID NO:13, 14, or 15, and fragments and variants thereof that encode polypeptides comprising AHAS activity.

The present disclosure provides AHASL proteins with amino acid substitutions at particular amino acid positions within conserved regions of the *Brassica* AHASL proteins disclosed herein. Unless otherwise indicated herein, particular amino acid positions refer to the position of that amino acid in the full-length *A. thaliana* AHASL amino acid sequences set forth in SEQ ID NO:1. Furthermore, those of ordinary skill will recognize that such amino acid positions can vary depending on whether amino acids are added to or removed from, for example, the N-terminal end of an amino acid sequence. Thus, the disclosure encompasses the amino substitutions at the recited position or equivalent position (e.g., "amino acid position 653 or equivalent position"). By "equivalent position" is intended to mean a position that is within the same conserved region as the exemplified amino acid position. For example, amino acid 122 in SEQ ID NO:1 is the equivalent position to amino acid 107 of SEQ ID NO:4 and the equivalent position to amino acid 104 of SEQ ID NO:5. Similarly, amino acid 653 in the Arabidopsis thaliana AHASL protein having the amino acid sequence set forth in SEQ ID NO:1 is the equivalent position to amino acid 638 in the Brassica AHASLIB and to amino acid 635 in the Brassica AHASLIA proteins having the amino acid sequence as set forth in SEQ ID NO:2 and 3 respectively.

Disclosed are isolated polynucleotide molecules and isolated polypeptides for *Brassica* AHASL proteins. The polynucleotide molecules of the disclosure comprise nucleotide sequences that encode herbicide-resistant AHASL proteins of the disclosure. The herbicide-resistant AHASL proteins of the disclosure comprise a polypeptide encoded by a nucleotide sequence selected from the group consisting of a) the nucleotide sequence as set forth in SEQ ID NO:13; b) the nucleotide sequence as set forth in SEQ ID NO:14; c) the nucleotide sequence as set forth in SEQ ID NO:15; d) a nucleotide sequence having at least 90% sequence identity to the nucleotide sequence as set forth in SEQ ID NO:13, wherein the protein has an asparagine at a position corresponding to position 653 of SEQ ID NO:1, or position 638 of SEQ ID NO:2, or position 635 of SEQ ID NO:3; e) a nucleotide sequence having at least 90% sequence identity to the nucleotide sequence as set forth in SEQ ID NO:14, wherein the protein has a threonine at a position corresponding to position 122 of SEQ ID NO:1, or position 107 of SEQ ID NO:4, or position 104 of SEQ ID NO:5; f) a nucleotide sequence having at least 90% sequence identity to the nucleotide sequence as set forth in SEQ ID NO:15, wherein the protein has a threonine at a position corresponding to position 122 of SEQ ID NO:1, or position 107 of SEQ ID NO:4, or position 104 of SEQ ID NO:5. The aforementioned AHASL protein further comprises at least one mutation selected from the group consisting of a) an asparagine at a position corresponding to position 653 of SEQ ID NO:1, or position 638 of SEQ ID NO:2, or position 635 of SEQ ID NO:3; b) a threonine at a position corresponding to position 122 of SEQ ID NO:1, or position 107 of SEQ ID NO:4, or position 104 of SEQ ID NO:5; and c) a leucine at a position corresponding to position 574 of SEQ ID NO:1, or position 556 of SEQ ID NO:6.

The disclosure encompasses isolated or substantially purified nucleic acid or protein compositions. An "isolated" or "purified" polynucleotide molecule or protein, or biologically active portion thereof, is substantially or essentially free from components that normally accompany or interact with the polynucleotide molecule or protein as found in its naturally occurring environment. Thus, an isolated or purified polynucleotide molecule or protein is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various aspects, the isolated polynucleotide molecule can contain less than about 5 kb, 4 kb, 3 kb, 2 kb, 1 kb, 0.5 kb, or 0.1 kb of nucleotide sequences that naturally flank the polynucleotide molecule in genomic DNA of the cell from which the nucleic acid is derived. A protein that is substantially free of cellular material includes preparations of protein having less than about 30%, 20%, 10%, 5%, or 1% (by dry weight) of contaminating protein. When the protein of the disclosure or biologically active portion thereof is recombinantly produced, preferably culture medium represents less than about 30%, 20%, 10%, 5%, or 1% (by dry weight) of chemical precursors or non-protein-of-interest chemicals.

The present disclosure provides isolated polypeptides comprising AHASLI proteins. The isolated polypeptides comprise an amino acid sequence selected from the group consisting of the amino acid sequence set forth in SEQ ID NO: 3, 4, or 5, the amino acid sequence encoded by the nucleotide sequence set forth in SEQ ID NO:13, 14, or 15, and functional fragments and variants of said amino acid sequences that encode an AHASLI polypeptide comprising AHAS activity. By "functional fragments and variants" is intended fragments and variants of the exemplified polypeptides that comprise AHAS activity.

In certain embodiments of the invention, the methods involve the use of herbicide-tolerant or herbicide-resistant plants. By an "herbicide-tolerant" or "herbicide-resistant" plant, it is intended that a plant that is tolerant or resistant to at least one herbicide at a level that would normally kill, or inhibit the growth of, a normal or wild-type plant. In one embodiment of the invention, the herbicide-tolerant **Brassica juncea** plants of the invention comprise an herbicide-tolerant or herbicide-resistant AHASL protein. By "herbicide-tolerant AHASL protein" or "herbicide-resistant AHASL protein", it is intended that such an AHASL protein displays higher AHAS activity, relative to the AHAS activity of a wild-type AHASL protein, when in the presence of at least one herbicide that is known to interfere with AHAS activity and at a concentration or level of the herbicide that is to known to inhibit the AHAS activity of the wild-type AHASL protein. Furthermore, the AHAS activity of such an herbicide-tolerant or herbicide-resistant AHASL protein may be referred to herein as "herbicide-tolerant" or "herbicide-resistant" AHAS activity.

For the present invention, the terms "herbicide-tolerant" and "herbicide-resistant" are used interchangeable and are intended to have an equivalent meaning and an equivalent scope. Similarly, the terms "herbicide-tolerance" and "herbicide-resistance" are used interchangeable and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively.

The disclosure encompasses herbicide-resistant AHASLI polynucleotides and herbicide-resistant AHASLI proteins. By "herbicide-resistant AHASLI polynucleotide" is intended a polynucleotide that encodes a protein comprising herbicide-resistant AHAS activity. By "herbicide-resistant AHASLI protein" is intended a protein or polypeptide that comprises herbicide-resistant AHAS activity.

Further, it is recognized that an herbicide-tolerant or herbicide-resistant AHASL protein can be introduced into a plant by transforming a plant or ancestor thereof with a nucleotide sequence encoding an herbicide-tolerant or herbicide-resistant AHASL protein. Such herbicide-tolerant or herbicide-resistant AHASL proteins are encoded by the herbicide-tolerant or herbicide-resistant *AHASL* polynucleotides. Alternatively, an herbicide-tolerant or herbicide-resistant AHASL protein may occur in a plant as a result of a naturally occurring or induced mutation in an endogenous *AHASL* gene in the genome of a plant or progenitor thereof.

The present invention provides Brassica juncea plants, plant tissues, plant cells, and host cells with increased and/or enhanced resistance or tolerance to at least one herbicide, particularly an herbicide that interferes with the activity of the AHAS enzyme, more particularly an imidazolinone or sulfonylurean herbicide. The term 'enhanced' refers to an increase in the amount of resistance or tolerance above that which is expected. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, microspore, or host cell, but that said amount does not kill or inhibit as severely the growth of the herbicide-resistant Brassica juncea plants, plant tissues, plant cells, microspores, and host cells of the present invention. Typically, the effective amount of an herbicide is an amount that is routinely used in agricultural production systems to kill weeds of interest. Such an amount is known to those of ordinary skill in the art, or can be easily determined using methods known in the art. Furthermore, it is recognized that the effective amount of an herbicide in an agricultural production system might be substantially different than an effective amount of an herbicide for a plant culture system such as, for example, the microspore culture system.

The herbicides of the present disclosure are those that interfere with the activity of the AHAS enzyme such that AHAS activity is reduced in the presence of the herbicide. Such herbicides may also be referred to herein as "AHAS-inhibiting herbicides" or simply "AHAS inhibitors." As used herein, an "AHAS-inhibiting herbicide" or an "AHAS inhibitor" is not meant to be limited to single herbicide that interferes with the activity of the AHAS enzyme. Thus, unless otherwise stated or evident from the context, an "AHAS-inhibiting herbicide" or an "AHAS inhibitor" can be a one herbicide or a mixture of two, three, four, or more herbicides, each of which interferes with the activity of the AHAS enzyme.

By "similar, wild-type, plant, plant tissue, plant cell or host cell" is intended a plant, plant tissue, plant cell, or host cell, respectively, that lacks the herbicide-resistance characteristics and/or particular polynucleotide that are disclosed herein. The use of the term "wild-type" is not, therefore, intended to imply that a plant, plant tissue, plant cell, or other host cell lacks recombinant DNA in its genome, and/or does not possess herbicide resistant characteristics that are different from those disclosed herein.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant including, plant calli, plant clumps, plant protoplasts and plant cell tissue cultures from which plants can be regenerated. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, an embryos, an ovule, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

The Brassica juncea plants of the present invention include non-transgenic plants . By "non-transgenic plant" is intended mean a plant lacking recombinant DNA in its genome. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

The present invention provides the herbicide-resistant *Brassica* line that is referred to herein as J04E-0122. A deposit of at least 2500 seeds from *Brassica* line J04E-0122 with the Patent Depository of the American Type Culture Collection (ATCC), Mansassas, VA 20110 USA was made on October 19, 2006 and assigned ATCC Patent Deposit Number PTA-7944. The present invention provides the herbicide-resistant *Brassica* line that is referred to herein as J04E-0130. A deposit of at least 2500 seeds from *Brassica* line J04E-0130 was made on October 19, 2006 and assigned ATCC Patent Deposit Number PTA-7945. The depositwill be maintained under the terms of the Budapest Treaty on the International Recognition of the Deposit of Microorganisms for the Purposes of Patent Procedure. The deposit of *Brassica* lines J04E-0122 and J04E-0130was made by BASF Plant Science, 26 Davis Drive, Research Triangle Park, North Carolina 27709, United States of America, for a term of at least 30 years and at least 5 years after the most recent request for the furnishing of a sample of the deposit is received by the ATCC. Additionally, Applicants have satisfied all the requirements of 37 C.F.R. §§ 1.801-1.809, including providing an indication of the viability of the sample.

The single mutant herbicide-resistant *Brassica* lines J04E-0122, and J04E-0130, of the present invention were produced by mutation breeding. Wild-type *Brassica* microspores were mutagenized by exposure to a mutagen, particularly a chemical mutagen, more particularly ethyl nitroso-urea (ENU). However, the present invention is not limited to herbicide-resistant *Brassica juncea* plants that are produced by a mutagenesis method involving the chemical mutagen ENU. Any mutagenesis method known in the art may be used to produce the herbicide-resistant *Brassica juncea* plants of the present invention. Such mutagenesis methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 250 to 290 nm), and chemical mutagens such as ethyl methanesulfonate (EMS), base analogues (e.g., 5-bromo-uracil), related compounds (e.g., 8-ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348. Further details of mutation breeding can be found in "Principals of Cultivar Development" Fehr, 1993 Macmillan Publishing Company.

Analysis of the AHASLI gene of the *Brassica* plant of the J04E-0130 line revealed a mutation that results in the substitution of a threonine for an alanine found at amino acid position 107 of the *B.juncea* AHASL gene on the B genome and confers enhanced resistance to an herbicide. Thus, the present invention discloses that substituting another amino acid for the alanine at position 107 (corresponding to amino acid 122 of the *A. thaliana* AHASLI) can cause a *Brassica* plant to have increased resistance to an herbicide, particularly an imidazolinone and/or sulfonylurean herbicide. The herbicide-resistant *Brassica juncea* plants of the invention include, but are not limited to those *Brassica juncea* plants which comprise in their genomes at least one copy of an AHASL1 polynucleotide that encodes an herbicide-resistant AHASL1 protein that comprises an threonine at amino acid position 107 or equivalent position.

Analysis of the AHASL1 gene of the *Brassica* plant of the J04E-0122 line revealed a mutation that results in the substitution of a threonine for an alanine found at amino acid position 104 of the *B.juncea* AHASL gene on the A genome and confers increased resistance to an herbicide. Thus, the present invention discloses that substituting another amino acid for the alanine at position 104 (corresponding to amino acid 122 of the A. *thaliana* AHASL1) can cause a *Brassica* plant to have increased resistance to an herbicide, particularly an imidazolinone and/or sulfonylurean herbicide. The herbicide-resistant *Brassica juncea* plants of the invention include, but are not limited to those *Brassica juncea* plants which comprise in their genomes at least one copy of an AHASL1 polynucleotide that encodes an herbicide-resistant AHASL1 protein that comprises an threonine at amino acid position 104 or equivalent position.

The present invention provides plants and methods of preparing AHAS herbicide resistant *Brassica juncea* plants, *Brassica juncea* plants having increased tolerance to AHAS herbicides, and seeds of such plants. Thus, the plants exemplified herein may be used in breeding programs to develop additional herbicide resistant *B.juncea* plants, such as commercial varieties of *B. juncea.* In accordance with such methods, a first *Brassica* parent plant may be used in crosses with a second *Brassica* parent plant, where at least one of the first or second *Brassica* parent plants contains at least one AHAS herbicide resistance mutation. One application of the process is in the production of F1 hybrid plants. Another important aspect of this process is that the process can be used for the development of novel parent, dihaploid or inbred lines. For example, a *Brassica* line as described herein could be crossed to any second plant, and the resulting hybrid progeny each selfed and/or sibbed for about 5 to 7 or more generations, thereby providing a large number of distinct, parent lines. These parent lines could then be crossed with other lines and the resulting hybrid progeny analyzed for beneficial characteristics. In this way, novel lines conferring desirable characteristics could be identified. Various breeding methods may be used in the methods, including haploidy, pedigree breeding, single-seed descent, modified single seed descent, recurrent selection, and backcrossing.

*Brassica* lines can be crossed by either natural or mechanical techniques. Mechanical pollination can be effected either by controlling the types of pollen that can be transferred onto the stigma or by pollinating by hand.

Descendent and/or progeny *Brassica* plants may be evaluated by any method to determine the presence of a mutated AHASL polynucleotide or polypeptide. Such methods include phenotypic evaluations, genotypic evaluations, or combinations thereof. The progeny *Brassica* plants may be evaluated in subsequent generations for herbicide resistance, and other desirable traits. Resistance to AHAS-inhibitor herbicides may be evaluated by exposing plants to one or more appropriate AHAS-inhibitor herbicides and evaluating herbicide injury. Some traits, such as lodging resistance and plant height, may be evaluated through visual inspection of the plants, while earliness of maturity may be evaluated by a visual inspection of seeds within pods (siliques). Other traits, such as oil percentage, protein percentage, and total glucosinolates of the seeds may be evaluated using techniques such as Near Infrared Spectroscopy and/or liquid chromatography and/or gas chromatography.

Genotypic evaluation of the *Brassica* plants includes using techniques such as Isozyme Electrophoresis, Restriction Fragment Length Polymorphisms (RFLPs), Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), Amplified Fragment Length Polymorphisms (AFLPs), Simple Sequence Repeats (SSRs) which are also referred to as "Microsatellites." Additional compositions and methods for analyzing the genotype of the *Brassica* plants provided herein include those methods disclosed in U.S. Publication No. 2004/0171027, U.S. Publication No. 2005/02080506, and U.S. Publication No. 2005/0283858.

Evaluation and manipulation (through exposure to one or more appropriate AHAS-inhibitor herbicides) may occur over several generations. The performance of the new lines may be evaluated using objective criteria in comparison to check varieties. Lines showing the desired combinations of traits are either crossed to another line or self-pollinated to produce seed. Self-pollination refers to the transfer of pollen from one flower to the same flower or another flower of the same plant. Plants that have been self-pollinated and selected for type for many generations become homozygous at almost all gene loci and produce a uniform population of true breeding progeny.

Any breeding method may be used in the methods of the present disclosure. In one example, the herbicide-resistant Brassica juncea plants of the present invention may be bred using a haploid method. In such methods, parents having the genetic basis for the desired complement of characteristics are crossed in a simple or complex cross. Crossing (or cross-pollination) refers to the transfer of pollen from one plant to a different plant. Progeny of the cross are grown and microspores (immature pollen grains) are separated and filtered, using techniques known to those skilled in the art [(e.g. Swanson, E. B. et al., "Efficient isolation of microspores and the production of microspore-derived embryos in Brassica napus, L. Plant Cell Reports, 6: 94-97 (1987); and Swanson, E. B., Microspore culture in Brassica, pp. 159-169 in Methods in Molecular Biology, vol. 6, Plant Cell and Tissue Culture, Humana Press, (1990)].

These microspores exhibit segregation of genes. The microspores are cultured in the presence of an appropriate AHAS-inhibitor herbicide, such as imazethapyr (e.g. PURSUIT.TM.) or imazamox (e.g. RAPTOR.TM.) or a 50/50 mix of imazethapyr and imazamox (e.g. ODYSSEY.TM.), which kills microspores lacking the mutations responsible for resistance to the herbicide. Microspores carrying the genes responsible for resistance to the herbicide survive and produce embryos, which form haploid plants. Their chromosomes are then doubled to produce doubled haploids.

Other breeding methods may also be used in accordance with the present invention. For example, pedigree breeding may be used for the improvement of largely self-pollinating crops such as Brassica and canola. Pedigree breeding starts with the crossing of two genotypes, each of which may have one or more desirable characteristics that is lacking in the other or which complements the other. If the two original parents do not provide all of the desired characteristics, additional parents can be included in the crossing plan.

These parents may be crossed in a simple or complex manner to produce a simple or complex F₁. An F2 population is produced from the F₁ by selfing one or several F1 plants, or by intercrossing two F₁'s (i.e., sib mating). Selection of the best individuals may begin in the F2 generation, and beginning in the F3 the best families, and the best individuals within the best families are selected. Replicated testing of families can begin in the F4 generation to improve the effectiveness of selection for traits with low heritability. At an advanced stage of inbreeding (i.e., F6 and F7), the best lines or mixtures of phenotypically similar lines may be tested for potential release as new cultivars. However, the pedigree method is more time-consuming than the haploidy method for developing improved AHAS-herbicide resistant plants, because the plants exhibit segregation for multiple generations, and the recovery of desirable traits is relatively low.

The single seed descent (SSD) procedure may also be used to breed improved varieties. The SSD procedure in the strict sense refers to planting a segregating population, harvesting a sample of one seed per plant, and using the population of single seeds to plant the next generation. When the population has been advanced from the F2 to the desired level of inbreeding, the plants from which lines are derived will each trace to different F2 individuals. The number of plants in a population declines each generation due to failure of some seeds to germinate or some plants to produce at least one seed. As a result, not all of the plants originally sampled in the F2 population will be represented by a progeny when generation advance is completed.

In a multiple-seed procedure, canola breeders commonly harvest one or more pods from each plant in a population and thresh them together to form a bulk. Part of the bulk is used to plant the next generation and part is put in reserve. The procedure has been referred to as modified single-seed descent or the pod-bulk technique. The multiple-seed procedure has been used to save labor at harvest. It is considerably faster to thresh pods with a machine than to remove one seed from each by hand for the single-seed procedure. The multiple-seed procedure also makes it possible to plant the same number of seeds of a population each generation of inbreeding. Enough seeds are harvested to make up for those plants that did not germinate or produce seed.

Backcross breeding can be used to transfer a gene or genes for a simply inherited, highly heritable trait from a source variety or line (the donor parent) into another desirable cultivar or inbred line (the recurrent parent). After the initial cross, individuals possessing the phenotype of the donor parent are selected and are repeatedly crossed (backcrossed) to the recurrent parent. When backcrossing is complete, the resulting plant is expected to have the attributes of the recurrent parent and the desirable trait transferred from the donor parent.

Improved varieties may also be developed through recurrent selection. A genetically variable population of heterozygous individuals is either identified or created by intercrossing several different parents. The best plants are selected based on individual superiority, outstanding progeny, or excellent combining ability. The selected plants are intercrossed to produce a new population in which further cycles of selection are continued.

In another aspect, the present disclosure provides a method of producing a Brassica plant having resistance to AHAS herbicides comprising: (a) crossing a first Brassica line with a second Brassica line to form a segregating population, where the first Brassica line is an AHAS herbicide resistant Brassica plant; (b) screening the population for increased AHAS herbicide resistance; and (c) selecting one or more members of the population having increased AHAS resistance relative to a wild-type Brassica plant.

In another aspect, the present disclosure provides a method of introgressing an AHAS herbicide resistance trait into a *Brassica* plant comprising: (a) crossing at least a first AHAS herbicide resistant *Brassica* line with a second *Brassica* line to form a segregating population; (b) screening the population for increased AHAS herbicide resistance; and (c) selecting at least one member of the population having increased AHAS herbicide resistance.

Alternatively, in another aspect of the disclosure, both first and second parent *Brassica* plants can be an AHAS herbicide resistant *Brassica* plant as described herein. Thus, any *Brassica* plant produced using a *Brassica* plant having increased AHAS herbicide resistance as described herein forms a part of the disclosure. As used herein, crossing can mean selfing, sibbing, backcrossing, crossing to another or the same parent line, crossing to populations, and the like.

The present disclosure also provides methods for producing an herbicide-resistant plant, particularly an herbicide-resistant *Brassica* plant, through conventional plant breeding involving sexual reproduction. The methods comprise crossing a first plant that is resistant to an herbicide to a second plant that is not resistant to the herbicide. The first plant can be any of the herbicide resistant plants of the present disclosure including, for example, transgenic plants comprising at least one of the polynucleotides of the present disclosure that encode an herbicide resistant AHASL and non-transgenic *Brassica* plants that comprise the herbicide-resistance characteristics of the *Brassica* plant of J04E-0130, or J04E-0122. The second plant can be any plant that is capable of producing viable progeny plants (i.e., seeds) when crossed with the first plant. Typically, but not necessarily, the first and second plants are of the same species. The methods of the disclosure can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the disclosure can additionally involve selecting plants that comprise the herbicide resistance characteristics of the first plant.

The present disclosure further provides methods for increasing the herbicide-resistance of a plant, particularly an herbicide-resistant *Brassica* plant, through conventional plant breeding involving sexual reproduction. The methods comprise crossing a first plant that is resistant to an herbicide to a second plant that may or may not be resistant to the herbicide or may be resistant to different herbicide or herbicides than the first plant. The first plant can be any of the herbicide resistant plants of the present disclosure including, for example, transgenic plants comprising at least one of the polynucleotides of the present disclosure that encode an herbicide resistant AHASL and non-transgenic *Brassica* plants that comprise the herbicide-resistance characteristics of the *Brassica* plant of J04E-0130, or J04E- 0122. The second plant can be any plant that is capable of producing viable progeny plants (i.e., seeds) when crossed with the first plant. Typically, but not necessarily, the first and second plants are of the same species; as well, the first and second plants can be from different species but within the same genus (example: *Brassica juncea x Brassica napus, Brassica juncea x Brassica rapa, Brassica juncea x Brassica oleracea, Brassica juncea × Brassica nigra,* etc.), and also, the first and second plants are of different genera (example: *Brassica × Sinapis).* The progeny plants produced by this method of the present disclosure have increased resistance to an herbicide when compared to either the first or second plant or both. When the first and second plants are resistant to different herbicides, the progeny plants will have the combined herbicide resistance characteristics of the first and second plants. The methods of the disclosure can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the disclosure can additionally involve selecting plants that comprise the herbicide resistance characteristics of the first plant, the second plant, or both the first and the second plant.

The Brassica juncea plants of the present invention can be non-transgenic. An example of a non-transgenic *Brassica juncea* plant having increased resistance to imidazolinone and/or sulfonylurean herbicides includes the *Brassica juncea* plant of J04E-0130, or J04E-0122;or a mutant, a recombinant, or a genetically engineered derivative of the plant of J04E-0130, or J04E-0122; or of any progeny of the plant of J04E-0130, or J04E-0122; or a plant that is a progeny of any of these plants; or a plant that comprises the herbicide resistance characteristics of the plant of J04E-0130, or J04E-0122.

The present disclosure also provides Brassica juncea plants, plant organs, plant tissues, plant cells, seeds, and non-human host cells that are transformed with at least one polynucleotide molecule, expression cassette, or transformation vector of the disclosure. Such transformed Brassica juncea plants, plant organs, plant tissues, plant cells, seeds, and non-human host cells have enhanced tolerance or resistance to at least one herbicide, at levels of the herbicide that kill or inhibit the growth of an untransformed Brassica juncea plant, plant tissue, plant cell, or non-human host cell, respectively.

The present invention also provides a seed of a *Brassica juncea* plant capable of producing a *Brassica juncea* plant having AHAS herbicide resistance obtained from *Brassica juncea* plants produced by the methods of the present disclosure.

In another aspect, the present invention also provides for a Brassica juncea plant grown from the seed of a *Brassica juncea* plant having AHAS herbicide resistance obtained from *Brassica juncea* plants grown for the seed having the herbicide resistance trait, as well as plant parts and tissue cultures from such plants.

Also provided herein is a container of *Brassica* seeds, where the seeds are capable of producing an AHAS herbicide resistant *Brassica* plant. The container of *Brassica* seeds may contain any number, weight or volume of seeds. For example, a container can contain at least, or greater than, about 10, 25, 50, 75, 100, 200, 300, 400, 500, 600, 700, 800, 900, 1000, 1500, 2000, 2500, 3000, 3500, 4000, 4500, 5000 or more seeds. Alternatively, the container can contain at least, or greater than, about 1 ounce, 5 ounces, 10, ounces, 1 pound, 2 pounds, 3 pounds, 4 pounds, 5 pounds or more seeds.

Containers of *Brassica* seeds may be any container available in the art. By way of non-limiting example, a container may be a box, a bag, a packet, a pouch, a tape roll, a pail, a foil, or a tube.

In another aspect, the seeds contained in the containers of *Brassica* seeds can be treated or untreated seeds. In one aspect, the seeds can be treated to improve germination, for example, by priming the seeds, or by disinfection to protect against seed-born pathogens. In another aspect, seeds can be coated with any available coating to improve, for example, plantability, seed emergence, and protection against seed-born pathogens. Seed coating can be any form of seed coating including, but not limited to pelleting, film coating, and encrustments.

The present disclosure also provides methods for increasing AHAS activity in a plant comprising transforming a plant with a polynucleotide construct comprising a promoter operably linked to an AHASL1 nucleotide sequence of the disclosure. The methods involve introducing a polynucleotide construct of the disclosure into at least one plant cell and regenerating a transformed plant therefrom. The polynucleotide construct comprises at least one nucleotide that encodes an herbicide-resistant AHASL protein of the disclosure, particularly the nucleotide sequence set forth in SEQ ID NO: 13, 14, or 15, nucleotide sequences encoding the amino acid sequence set forth in SEQ ID NO: 3, 4 or 5, and fragments and variants thereof. The methods further involve the use of a promoter that is capable of driving gene expression in a plant cell. Preferably, such a promoter is a constitutive promoter or a tissue-preferred promoter. A plant produced by this method comprises increased AHAS activity, particularly herbicide-tolerant AHAS activity, when compared to an untransformed plant. Thus, the methods find use in enhancing or increasing the resistance of a plant to at least one herbicide that interferes with the catalytic activity of the AHAS enzyme, particularly an imidazolinone herbicide.

The present disclosure provides a method for producing an herbicide-resistant plant comprising transforming a plant cell with a polynucleotide construct comprising a nucleotide sequence operably linked to a promoter that drives expression in a plant cell and regenerating a transformed plant from said transformed plant cell. The nucleotide sequence is selected from those nucleotide sequences that encode the herbicide-resistant AHASL proteins of the disclosure, particularly the nucleotide sequence set forth in SEQ ID NO:13, 14, or 15, nucleotide sequences encoding the amino acid sequence set forth in SEQ ID NO:3, 4, or 5, and fragments and variants thereof. An herbicide-resistant plant produced by this method comprises enhanced resistance, compared to an untransformed plant, to at least one herbicide, particularly an herbicide that interferes with the activity of the AHAS enzyme such as, for example, an imidazolinone herbicide or a sulfonylurean herbicide.

The present disclosure provides expression cassettes for expressing the polynucleotide molecules of the disclosure in plants, plant cells, and other, non-human host cells. The expression cassettes comprise a promoter expressible in the plant, plant cell, or other host cells of interest operably linked to a polynucleotide molecule of the disclosure that encodes an herbicide-resistant AHASL protein. If necessary for targeting expression to the chloroplast, the expression cassette can also comprise an operably linked chloroplast-targeting sequence that encodes of a chloroplast transit peptide to direct an expressed AHASL protein to the chloroplast.

The expression cassettes of the disclosure find use in a method for enhancing the herbicide tolerance of a plant or a host cell. The method involves transforming the plant or host cell with an expression cassette of the disclosure, wherein the expression cassette comprises a promoter that is expressible in the plant or host cell of interest and the promoter is operably linked to a polynucleotide of the disclosure that comprises a nucleotide sequence encoding an herbicide-resistant AHASL1 protein of the disclosure. The method further comprises regenerating a transformed plant from the transformed plant cell.

The use of the term "polynucleotide constructs" herein is not intended to limit the present disclosure to polynucleotide constructs comprising DNA. Those of ordinary skill in the art will recognize that polynucleotide constructs, particularly polynucleotides and oligonucleotides, comprised of ribonucleotides and combinations of ribonucleotides and deoxyribonucleotides may also be employed in the methods disclosed herein. Thus, the polynucleotide constructs of the present disclosure encompass all polynucleotide constructs that can be employed in the methods of the present disclosure for transforming plants including, but not limited to, those comprised of deoxyribonucleotides, ribonucleotides, and combinations thereof. Such deoxyribonucleotides and ribonucleotides include both naturally occurring molecules and synthetic analogues. The polynucleotide constructs of the disclosure also encompass all forms of polynucleotide constructs including, but not limited to, single-stranded forms, double-stranded forms, hairpins, stem-and-loop structures, and the like. Furthermore, it is understood by those of ordinary skill the art that each nucleotide sequences disclosed herein also encompasses the complement of that exemplified nucleotide sequence.

Furthermore, it is recognized that the methods of the disclosure may employ a polynucleotide construct that is capable of directing, in a transformed plant, the expression of at least one protein, or at least one RNA, such as, for example, an antisense RNA that is complementary to at least a portion of an mRNA. Typically such a polynucleotide construct is comprised of a coding sequence for a protein or an RNA operably linked to 5' and 3' transcriptional regulatory regions. Alternatively, it is also recognized that the methods of the disclosure may employ a polynucleotide construct that is not capable of directing, in a transformed plant, the expression of a protein or an RNA.

Further, it is recognized that, for expression of a polynucleotides of the disclosure in a host cell of interest, the polynucleotide is typically operably linked to a promoter that is capable of driving gene expression in the host cell of interest. The methods of the disclosure for expressing the polynucleotides in host cells do not depend on particular promoter. The methods encompass the use of any promoter that is known in the art and that is capable of driving gene expression in the host cell of interest.

The present disclosure encompasses AHASL1 polynucleotide molecules and fragments and variants thereof. Polynucleotide molecules that are fragments of these nucleotide sequences are also encompassed by the present disclosure. By "fragment" is intended a portion of the nucleotide sequence encoding an AHASL1 protein of the disclosure. A fragment of an AHASL1 nucleotide sequence of the disclosure may encode a biologically active portion of an AHASL1 protein, or it may be a fragment that can be used as a hybridization probe or PCR primer using methods disclosed below. A biologically active portion of an AHASL1 protein can be prepared by isolating a portion of one of the AHASL1 nucleotide sequences of the disclosure, expressing the encoded portion of the AHASL1 protein (e.g., by recombinant expression *in vitro),* and assessing the activity of the encoded portion of the AHASL1 protein. Polynucleotide molecules that are fragments of an AHASL1 nucleotide sequence comprise at least about 15,20, 50, 75, 100, 200, 300, 350, 400, 450, 500, 550, 600, 650, 700, 750, 800, 850, or 900 nucleotides, or up to the number of nucleotides present in a full-length nucleotide sequence disclosed herein depending upon the intended use.

A fragment of an AHASL1 nucleotide sequence that encodes a biologically active portion of an AHASL1 protein of the disclosure will encode at least about 15, 25, 30, 50, 75, 100, 125, 150, 175, 200, 225, or 250 contiguous amino acids, or up to the total number of amino acids present in a full-length AHASL1 protein of the disclosure. Fragments of an AHASL1 nucleotide sequence that are useful as hybridization probes for PCR primers generally need not encode a biologically active portion of an AHASL1 protein.

Polynucleotide molecules that are variants of the nucleotide sequences disclosed herein are also encompassed by the present disclosure. "Variants" of the AHASL1 nucleotide sequences of the disclosure include those sequences that encode the AHASL1 proteins disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHASL1 protein disclosed in the present disclosure as discussed below. Generally, nucleotide sequence variants of the disclosure will have at least about 75%, 80%, 85%, 90%, 91%, 92%, 93%, 94%, 95%, 96%, 97%, 98%, or 99% identity to a particular nucleotide sequence disclosed herein. A variant AHASL1 nucleotide sequence will encode an AHASL1 protein, respectively, that has an amino acid sequence having at least about 75%, 80%, 85%, 90%, 91%, 92%, 93%, 94%, 95%, 96%, 97%, 98%, or 99% identity to the amino acid sequence of an AHASL1 protein disclosed herein.

In addition, the skilled artisan will further appreciate that changes can be introduced by mutation into the nucleotide sequences of the disclosure thereby leading to changes in the amino acid sequence of the encoded AHASL1 proteins without altering the biological activity of the AHASL1 proteins. Thus, an isolated polynucleotide molecule encoding an AHASL1 protein having a sequence that differs from that of SEQ ID NO: 11 can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present disclosure.

For example, preferably, conservative amino acid substitutions may be made at one or more predicted, nonessential amino acid residues. A "nonessential" amino acid residue is a residue that can be altered from the wild-type sequence of an AHASL1 protein (e.g., the sequence of SEQ ID NO: 1) without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Such substitutions would not be made for conserved amino acid residues, or for amino acid residues residing within a conserved motif.

The proteins of the disclosure may be altered in various ways including amino acid substitutions, deletions, truncations, and insertions. Methods for such manipulations are generally known in the art. For example, amino acid sequence variants of the AHASL1 proteins can be prepared by mutations in the DNA. Methods for mutagenesis and nucleotide sequence alterations are well known in the art. See, for example, Kunkel (1985) Proc. Natl. Acad. Sci. USA 82:488-492; Kunkel et al. (1987) Methods in Enzymol. 154:367-382; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be preferable.

Alternatively, variant AHASL1 nucleotide sequences can be made by introducing mutations randomly along all or part of an AHASL1 coding sequence, such as by saturation mutagenesis, and the resultant mutants can be screened for AHAS activity to identify mutants that retain AHAS activity, including herbicide-resistant AHAS activity. Following mutagenesis, the encoded protein can be expressed recombinantly, and the activity of the protein can be determined using standard assay techniques.

Thus, the nucleotide sequences of the disclosure include the sequences disclosed herein as well as fragments and variants thereof. The AHASL1 nucleotide sequences of the disclosure, and fragments and variants thereof, can be used as probes and/or primers to identify and/or clone *AHASL* homologues in other plants. Such probes can be used to detect transcripts or genomic sequences encoding the same or identical proteins.

In this manner, methods such as PCR, hybridization, and the like can be used to identify such sequences having substantial identity to the sequences of the disclosure. *See,* for example, Sambrook et al. (1989) Molecular Cloning: Laboratory Manual (2d ed., Cold Spring Harbor Laboratory Press, Plainview, NY) and Innis, et al. (1990) PCR Protocols: A Guide to Methods and Applications (Academic Press, NY). *AHASL* nucleotide sequences isolated based on their sequence identity to the AHASL1 nucleotide sequences set forth herein or to fragments and variants thereof are encompassed by the present disclosure.

In a hybridization method, all or part of a known AHASL1 nucleotide sequence can be used to screen cDNA or genomic libraries. Methods for construction of such cDNA and genomic libraries are generally known in the art and are disclosed in Sambrook et al. (1989) Molecular Cloning: A Laboratory Manual (2d ed., Cold Spring Harbor Laboratory Press, Plainview, NY). The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known AHASL1 nucleotide sequence disclosed herein. Degenerate primers designed on the basis of conserved nucleotides or amino acid residues in a known AHASL1 nucleotide sequence or encoded amino acid sequence can additionally be used. The probe typically comprises a region of nucleotide sequence that hybridizes under stringent conditions to at least about 12, preferably about 25, more preferably about 50, 75, 100, 125, 150, 175, 200, 250, 300, 350, 400, 500, 600, 700, 800, or 900 consecutive nucleotides of an AHASL1 nucleotide sequence of the disclosure or a fragment or variant thereof. Preparation of probes for hybridization is generally known in the art and is disclosed in Sambrook et al. (1989) Molecular Cloning: A Laboratory Manual (2d ed., Cold Spring Harbor Laboratory Press, Plainview, New York).

For example, the entire AHASL1 sequence disclosed herein, or one or more portions thereof, may be used as a probe capable of specifically hybridizing to corresponding AHASL1 sequences and messenger RNAs. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1× SSC at 60 to 65°C. Optionally, wash buffers may comprise about 0.1% to about 1% SDS. The duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) +0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with >90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology- Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Plainview, New York).

It is recognized that the polynucleotide molecules and proteins of the disclosure encompass polynucleotide molecules and proteins comprising a nucleotide or an amino acid sequence that is sufficiently identical to the nucleotide sequence of SEQ ID NOS:13, 14, and/or 15, or to the amino acid sequence of SEQ ID NOS:3, 4, and/or 5. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences that contain a common structural domain having at least about 45%, 55%, or 65% identity, preferably 75% identity, more preferably 85%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences *(i.e.,* percent identity = number of identical positions/total number of positions (e.g., overlapping positions) × 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the NBLAST and XBLAST programs of Altschul et al. (1990) J. Mol. Biol. 215:403. BLAST nucleotide searches can be performed with the NBLAST program, score = 100, wordlength = 12, to obtain nucleotide sequences homologous to the polynucleotide molecules of the disclosure. BLAST protein searches can be performed with the XBLAST program, score = 50, wordlength = 3, to obtain amino acid sequences homologous to protein molecules of the disclosure. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. *See* Altschul *et al.* (1997) *supra.* When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarity values provided herein refer to the value obtained using the full-length sequences of the disclosure and using multiple alignment by mean of the algorithm Clustal W (Nucleic Acid Research, 22(22):4673- 4680, 1994) using the program AlignX included in the software package Vector NTI Suite Version 9 (Invitrogen, Carlsbad, CA, USA) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Suite Version 9.

The AHASL1 nucleotide sequences of the disclosure include both the naturally occurring sequences as well as mutant forms, particularly mutant forms that encode AHASL1 proteins comprising herbicide-resistant AHAS activity. Likewise, the proteins of the disclosure encompass both naturally occurring proteins as well as variations and modified forms thereof. Such variants will continue to possess the desired AHAS activity. Obviously, the mutations that will be made in the DNA encoding the variant must not place the sequence out of reading frame and preferably will not create complementary regions that could produce secondary mRNA structure. See, EP Patent Application Publication No. 75,444.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by AHAS activity assays. *See,* for example, Singh et al. (1988) Anal. Biochem. 171:173-179.

Variant nucleotide sequences and proteins also encompass sequences and proteins derived from a mutagenic and recombinogenic procedure such as DNA shuffling. With such a procedure, one or more different AHASL coding sequences can be manipulated to create a new AHASL protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the AHASL1 gene of the disclosure and other known *AHASL* genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

The nucleotide sequences of the disclosure can be used to isolate corresponding sequences from other organisms, particularly other plants, more particularly other dicots. In this manner, methods such as PCR, hybridization, and the like can be used to identify such sequences based on their sequence homology to the sequences set forth herein. Sequences isolated based on their sequence identity to the entire AHASL1 sequences set forth herein or to fragments thereof are encompassed by the present disclosure. Thus, isolated sequences that encode for an AHASL protein and which hybridize under stringent conditions to the sequence disclosed herein, or to fragments thereof, are encompassed by the present disclosure.

In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

The AHASL1 polynucleotide sequences of the disclosure are provided in expression cassettes for expression in the plant of interest. The cassette will include 5' and 3' regulatory sequences operably linked to an AHASL1 polynucleotide sequence of the disclosure. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the AHASL1 polynucleotide sequence to be under the transcriptional regulation of the regulatory regions. The expression cassette may additionally contain selectable marker genes.

The expression cassette will include in the 5'-3' direction of transcription, a transcriptional and translational initiation region (i.e., a promoter), an AHASL1 polynucleotide sequence of the disclosure, and a transcriptional and translational termination region (i.e., termination region) functional in plants. The promoter may be native or analogous, or foreign or heterologous, to the plant host and/or to the AHASL1 polynucleotide sequence of the disclosure. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the AHASL1 polynucleotide sequence of the disclosure, it is intended that the promoter is not the native or naturally occurring promoter for the operably linked AHASL1 polynucleotide sequence of the disclosure. As used herein, a chimeric gene comprises a coding sequence operably linked to a transcription initiation region that is heterologous to the coding sequence.

While it may be preferable to express the AHASL1 polynucleotides of the disclosure using heterologous promoters, the native promoter sequences may be used. Such constructs would change expression levels of the AHASL1 protein in the plant or plant cell. Thus, the phenotype of the plant or plant cell is altered.

The termination region may be native with the transcriptional initiation region, may be native with the operably linked AHASL1 sequence of interest, may be native with the plant host, or may be derived from another source (i.e., foreign or heterologous to the promoter, the AHASL1 polynucleotide sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272*;* Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

Nucleotide sequences for enhancing gene expression can also be used in the plant expression vectors. These include the introns of the maize Adhl, intronl gene (Callis et al. Genes and Development 1:1183-1200, 1987), and leader sequences, (W-sequence) from the Tobacco Mosaic virus (TMV), Maize Chlorotic Mottle Virus and Alfalfa Mosaic Virus (Gallie et al. Nucleic Acid Res. 15:8693-8711, 1987 and Skuzeski et al. Plant Mol. Biol. 15:65-79, 1990). The first intron from the shrunkent-1 locus of maize, has been shown to increase expression of genes in chimeric gene constructs. U.S. Pat. Nos. 5,424,412 and 5,593,874 disclose the use of specific introns in gene expression constructs, and Gallie et al. (Plant Physiol. 106:929-939, 1994) also have shown that introns are useful for regulating gene expression on a tissue specific basis. To further enhance or to optimize AHAS small subunit gene expression, the plant expression vectors of the disclosure may also contain DNA sequences containing matrix attachment regions (MARs). Plant cells transformed with such modified expression systems, then, may exhibit overexpression or constitutive expression of a nucleotide sequence of the disclosure.

The expression cassettes may additionally contain 5' leader sequences in the expression cassette construct. Such leader sequences can act to enhance translation. Translation leaders are known in the art and include: picomavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) *(*Virology 154:9-20), and human immunoglobulin heavy-chain binding protein (BiP) (Macejak etal. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385*).* See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the disclosure. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810- 812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to target enhanced AHASL1 expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J 4(3):495-505. Such promoters can be modified, if necessary, for weak expression.

In one embodiment, the nucleic acids of interest are targeted to the chloroplast for expression. In this manner, where the nucleic acid of interest is not directly inserted into the chloroplast, the expression cassette will additionally contain a chloroplast-targeting sequence comprising a nucleotide sequence that encodes a chloroplast transit peptide to direct the gene product of interest to the chloroplasts. Such transit peptides are known in the art. With respect to chloroplast-targeting sequences, "operably linked" means that the nucleic acid sequence encoding a transit peptide *(i.e.,* the chloroplast-targeting sequence) is linked to the *AHASL* polynucleotide of the disclosure such that the two sequences are contiguous and in the same reading frame. See, for example, Von Heijne et al. (1991) Plant Mol. Biol. Rep. 9:104-126; Clark et al. (1989) J Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the AHASL1 proteins of the disclosure include a native chloroplast transit peptide, any chloroplast transit peptide known in art can be fused to the amino acid sequence of a mature AHASL1 protein of the disclosure by operably linking a chloroplast-targeting sequence to the 5'-end of a nucleotide sequence encoding a mature AHASL1 protein of the disclosure.

Chloroplast targeting sequences are known in the art and include the chloroplast small subunit of ribulose-1,5-bisphosphate carboxylase (Rubisco) (de Castro Silva Filho et al. ( 1996) Plant Mol. Biol. 30:769-780; Schnell et al. (1991) J. Biol Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol Chem. 264: 17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

As disclosed herein, the AHASL1 nucleotide sequences of the disclosure find use in enhancing the herbicide tolerance of plants that comprise in their genomes a gene encoding an herbicide-tolerant AHASL1 protein. Such a gene may be an endogenous gene or a transgene. Additionally, in certain aspects, the nucleic acid sequences of the present disclosure can be stacked with any combination of polynucleotide sequences of interest in order to create plants with a desired phenotype. For example, the polynucleotides of the present disclosure may be stacked with any other polynucleotides encoding polypeptides having pesticidal and/or insecticidal activity, such as, for example, the *Bacillus thuringiensis* toxin proteins (described in U.S. Patent Nos. 5,366,892; 5,747,450; 5,737,514; 5,723,756; 5,593,881; and Geiser et al. (1986) Gene 48:109). The combinations generated can also include multiple copies of any one of the polynucleotides of interest.

It is recognized that with these nucleotide sequences, antisense constructions, complementary to at least a portion of the messenger RNA (mRNA) for the AHASL1 polynucleotide sequences can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, preferably 80%, more preferably 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

The nucleotide sequences of the present disclosure may also be used in the sense orientation to suppress the expression of endogenous genes in plants. Methods for suppressing gene expression in plants using nucleotide sequences in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, preferably greater than about 65% sequence identity, more preferably greater than about 85% sequence identity, most preferably greater than about 95% sequence identity. See, U.S. Patent Nos. 5,283,184 and 5,034,323.

While the herbicide-resistant AHASL1 polynucleotides of the disclosure find use as selectable marker genes for plant transformation, the expression cassettes of the disclosure can include another selectable marker gene for the selection of transformed cells. Selectable marker genes, including those of the present disclosure, are utilized for the selection of transformed cells or tissues. Marker genes include, but are not limited to, genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Ce//48:555-566; Brown etal. (1987) Ce//49:603-612; Figge etal. (1988) Cell 52:713-722; Deuschle et al. (1989) Proc. Natl. Acad. Aci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb etal. (1991) Antimicrob. Agents Chemother. 35:1591-1595; Kleinschnidt etal. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724.

The above list of selectable marker genes is not meant to be limiting. Any selectable marker gene can be used in the present disclosure.

The isolated polynucleotide molecules comprising nucleotide sequence that encode the AHASL1 proteins of the disclosure can be used in vectors to transform plants so that the plants created have enhanced resistant to herbicides, particularly imidazolinone herbicides. The isolated AHASL1 polynucleotide molecules of the disclosure can be used in vectors alone or in combination with a nucleotide sequence encoding the small subunit of the AHAS (AHASS) enzyme in conferring herbicide resistance in plants. *See,* U.S. Patent No. 6,348,643.

Thus, the present disclosure provides transformation vectors comprising a selectable marker gene of the disclosure. The selectable marker gene comprises a promoter that drives expression in a host cell operably linked to a polynucleotide comprising a nucleotide sequence that encodes an herbicide-resistant AHASL protein of the disclosure. The transformation vector can additionally comprise a gene of interest to be expressed in the host cell and can also, if desired, include a chloroplast-targeting sequence that is operably linked to the polynucleotide of the disclosure.

The present disclosure further provides methods for using the transformation vectors of the disclosure to select for cells transformed with the gene of interest. Such methods involve the transformation of a host cell with the transformation vector, exposing the cell to a level of an imidazolinone or sulfonylurean herbicide that would kill or inhibit the growth of a non-transformed host cell, and identifying the transformed host cell by its ability to grow in the presence of the herbicide. In one embodiment of the disclosure, the host cell is a plant cell and the selectable marker gene comprises a promoter that drives expression in a plant cell.

The transformation vectors of the disclosure can be used to produce plants transformed with a gene of interest. The transformation vector will comprise a selectable marker gene of the disclosure and a gene of interest to be introduced and typically expressed in the transformed plant. Such a selectable marker gene comprises an herbicide-resistant AHASL1 polynucleotide of the disclosure operably linked to a promoter that drives expression in a host cell. For use in plants and plant cells, the transformation vector comprises a selectable marker gene comprising an herbicide-resistant AHASL1 polynucleotide of the disclosure operably linked to a promoter that drives expression in a plant cell.

The disclosure also relates to a plant expression vector comprising a promoter that drives expression in a plant operably linked to an isolated polynucleotide molecule of the disclosure. The isolated polynucleotide molecule comprises a nucleotide sequence encoding an AHASL1 protein, particularly an AHASL1 protein comprising an amino sequence that is set forth in SEQ ID NO: 2, 3, 4, 5, or 6, or a functional fragment and variant thereof. The plant expression vector of the disclosure does not depend on a particular promoter, only that such a promoter is capable of driving gene expression in a plant cell. Preferred promoters include constitutive promoters and tissue-preferred promoters.

The genes of interest of the disclosure vary depending on the desired outcome. For example, various changes in phenotype can be of interest including modifying the fatty acid composition in a plant, altering the amino acid content of a plant, altering a plant's insect and/or pathogen defense mechanisms, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

In one embodiment of the disclosure, the genes of interest include insect resistance genes such as, for example, *Bacillus thuringiensis* toxin protein genes (U.S. Patent Nos. 5,366,892; 5,747,450; 5,736,514; 5,723,756; 5,593,881; and Geiser et al. (1986) Gene 48:109).

The AHASL1 proteins or polypeptides of the disclosure can be purified from, for example, *Brassica* plants and can be used in compositions. Also, an isolated polynucleotide molecule encoding an AHASL1 protein of the disclosure can be used to express an AHASL1 protein of the disclosure in a microbe such as *E. coli* or a yeast. The expressed AHASL1 protein can be purified from extracts of *E. coli* or yeast by any method known to those or ordinary skill in the art.

The disclosure also relates to a method for creating a transgenic plant that is resistant to herbicides, comprising transforming a plant with a plant expression vector comprising a promoter that drives expression in a plant operably linked to an isolated polynucleotide molecule of the disclosure. The isolated polynucleotide molecule comprises a nucleotide sequence encoding an AHASL1 protein of the disclosure, particularly an AHASL1 protein comprising: the amino acid sequence that is set forth in SEQ ID NO:2, 3, 4, 5, or 6, the amino acid sequence encoded by SEQ ID NO:12, 13, 14, 15, or 16, or a functional fragment and variant of said amino acid sequences.

The disclosure also relates to the non-transgenic *Brassica* plants, transgenic plants produced by the methods of the disclosure, and progeny and other descendants of such non-transgenic and transgenic plants, which plants exhibit enhanced or increased resistance to herbicides that interfere with the AHAS enzyme, particularly imidazolinone and sulfonylurea herbicides.

The AHASL1 polynucleotides of the disclosure, particularly those encoding herbicide-resistant AHASL1 proteins, find use in methods for enhancing the resistance of herbicide-tolerant plants. In one embodiment of the disclosure, the herbicide-tolerant plants comprise an herbicide-tolerant or herbicide resistant AHASL protein. The herbicide-tolerant plants include both plants transformed with an herbicide-tolerant AHASL nucleotide sequences and plants that comprise in their genomes an endogenous gene that encodes an herbicide-tolerant AHASL protein. Such an herbicide-tolerant plant can be an herbicide-tolerant plant that has been genetically engineered for herbicide-tolerance or an herbicide-tolerant plant that was developed by means that do not involve recombinant DNA such as, for example, the *Brassica* plants of the present disclosure. Nucleotide sequences encoding herbicide-tolerant AHASL proteins and herbicide-tolerant plants comprising an endogenous gene that encodes an herbicide-tolerant AHASL protein include the polynucleotides and plants of the present disclosure and those that are known in the art. *See,* for example, U.S. Patent Nos. 5,013,659, 5,731,180, 5,767,361, 5,545,822, 5,736,629, 5,773,703, 5,773,704, 5,952,553 and 6,274,796. Such methods for enhancing the resistance of herbicide-tolerant plants comprise transforming an herbicide-tolerant plant with at least one polynucleotide construct comprising a promoter that drives expression in a plant cell that is operably linked to an herbicide resistant AHASL1 polynucleotide of the disclosure, particularly the polynucleotide encoding an herbicide-resistant AHASL1 protein set forth in SEQ ID NO:12, 13, 14, 15, or 16, polynucleotides encoding the amino acid sequence set forth in SEQ ID NO:2" 3, 4, 5, or 6 and fragments and variants of said polynucleotides that encode polypeptides comprising herbicide-resistant AHAS activity. A plant produced by this method has enhanced resistance to at least one herbicide, when compared to the herbicide-resistant plant prior to transformation with the polynucleotide construct of the disclosure.

Numerous plant transformation vectors and methods for transforming plants are available. *See,* for example, An, G. et al. (1986) Plant Pysiol., 81:301-305; Fry, J., et al. (1987) Plant Cell Rep. 6:321-325; Block, M. (1988) Theor. Appl Genet.76:767-774; Hinchee, et al. (1990) Stadler. Genet. Symp.203212.203-212; Cousins, et al. (1991) Aust. J. Plant Physiol. 18:481-494; Chee, P. P. and Slightom, J. L. (1992) Gene. 118:255-260; Christou, et al. (1992) Trends. Biotechnol. 10:239-246; D'Halluin, et al. (1992) Bio/Technol. 10:309-314; Dhir, et al. (1992) Plant Physiol. 99:81-88; Casas et al. (1993) Proc. Nat. Acad Sci. USA 90:11212-11216; Christou, P. (1993) In Vitra Cell. Dev. Biol.-Plant; 29P: 119-124; Davies, et al. (1993) Plant Cell Rep. 12:180-183; Dong, J. A. and Mchughen, A. (1993) Plant Sci. 91:139-148; Franklin, C. I. and Trieu, T. N. (1993) Plant. Physiol. 102:167; Golovkin, et al. (1993) Plant Sci. 90:41-52; Guo Chin Sei. Bull. 38:2072-2078; Asano, et al. (1994) Plant Cell Rep. 13; Ayeres N. M. and Park, W. D. (1994) Crit. Rev. Plant. Sci. 13:219-239; Barcelo, et al. (1994) Plant. J. 5:583-592; Becker, et al. (1994) Plant. J. 5:299-307; Borkowska et al. (1994) Acta. Physiol Plant. 16:225-230; Christou, P. (1994) Agro. Food. Ind. Hi Tech. 5: 17-27; Eapen et al. (1994) Plant Cell Rep. 13:582-586; Hartman, et al. (1994) Bio-Technology 12: 919923; Ritala, et al. (1994) Plant. Mol. Biol. 24:317-325; and Wan, Y. C. and Lemaux, P. G. (1994) Plant Physiol. 104:3748.

The methods of the disclosure involve introducing a polynucleotide construct into a plant. By "introducing" is intended presenting to the plant the polynucleotide construct in such a manner that the construct gains access to the interior of a cell of the plant. The methods of the disclosure do not depend on a particular method for introducing a polynucleotide construct to a plant, only that the polynucleotide construct gains access to the interior of at least one cell of the plant. Methods for introducing polynucleotide constructs into plants are known in the art including, but not limited to, stable transformation methods, transient transformation methods, and virus-mediated methods.

By "stable transformation" is intended that the polynucleotide construct introduced into a plant integrates into the genome of the plant and is capable of being inherited by progeny thereof. By "transient transformation" is intended that a polynucleotide construct introduced into a plant does not integrate into the genome of the plant.

For the transformation of plants and plant cells, the nucleotide sequences of the disclosure are inserted using standard techniques into any vector known in the art that is suitable for expression of the nucleotide sequences in a plant or plant cell. The selection of the vector depends on the preferred transformation technique and the target plant species to be transformed. In an embodiment of the disclosure, an AHASL1 nucleotide sequence is operably linked to a plant promoter that is known for high-level expression in a plant cell, and this construct is then introduced into a plant that that is susceptible to an imidazolinone herbicide and a transformed plant it regenerated. The transformed plant is tolerant to exposure to a level of an imidazolinone herbicide that would kill or significantly injure an untransformed plant. This method can be applied to any plant species; however, it is most beneficial when applied to crop plants, particularly crop plants that are typically grown in the presence of at least one herbicide, particularly an imidazolinone herbicide.

Methodologies for constructing plant expression cassettes and introducing foreign nucleic acids into plants are generally known in the art and have been previously described. For example, foreign DNA can be introduced into plants, using tumor-inducing (Ti) plasmid vectors. *Agrobacterium* based transformation techniques are well known in the art. The Agrobacterium strain *(e.g., Agrobacterium tumefaciens* or *Agrobacterium rhizogenes)* comprises a plasmid (Ti or Ri plasmid) and a T-DNA element which is transferred to the plant following infection with *Agrobacterium.* The T-DNA (transferred DNA) is integrated into the genome of the plant cell. The T-DNA may be localized on the Ri- or Ti-plasmid or is separately comprised in a so-called binary vector. Methods for the *Agrobacterium-mediated* transformation are described, for example, in Harsch RB et al. (1985) Science 225:1229f. The *Agrobacterium-*mediated transformation can be used in both dicotyledonous plants and monocotyledonous plants. The transformation of plants by Agrobacteria is described in White FF, Vectors for Gene Transfer in Higher Plants; in Transgenic Plants, Vol. 1, Engineering and Utilization, edited by S.D. Kung and R. Wu, Academic Press, 1993, pp. 15 - 38; Jenes B et al. (1993) Techniques for Gene Transfer, in: Transgenic Plants, Vol. 1, Engineering and Utilization, edited by S.D. Kung and R. Wu, Academic Press, pp. 128-143; Potrykus (1991) Annu Rev Plant Physiol Plant Molec Biol 42:205- 225. Other methods utilized for foreign DNA delivery involve the use of PEG mediated protoplast transformation, electroporation, microinjection whiskers, and biolistics or microprojectile bombardment for direct DNA uptake. Such methods are known in the art. (U.S. Pat. No. 5,405,765 to Vasil et al.*;* Bilang et al. (1991) Gene 100: 247-250; Scheid et al., (1991) Mol. Gen. Genet., 228: 104-112; Guerche et al., (1987) Plant Science 52: 111-116; Neuhause et al., (1987) Theor. Appl Genet. 75: 30-36; Klein et al., (1987) Nature 327: 70-73; Howell et al., (1980) Science 208:1265; Horsch et al., (1985) Science 227: 1229-1231; DeBlock et al., (1989) Plant Physiology 91: 694-701; Methods for Plant Molecular Biology (Weissbach and Weissbach, eds.) Academic Press, Inc. (1988) and Methods in Plant Molecular Biology (Schuler and Zielinski, eds.) Academic Press, Inc. (1989). The method of transformation depends upon the plant cell to be transformed, stability of vectors used, expression level of gene products and other parameters.

Other suitable methods of introducing nucleotide sequences into plant cells and subsequent insertion into the plant genome include microinjection as Crossway et al. (1986) Biotechniques 4:320-334, electroporation as described by Riggs et al. (1986) Proc. Natl. Acad. Sci. USA 83:5602-5606, *Agrobacterium-mediated* transformation as described by Townsend *et al.,* U.S. Patent No. 5,563,055, Zhao et al*,* U.S. Patent No. 5,981,840, direct gene transfer as described by Paszkowski et al. (1984) EMBO J. 3:2717-2722, and ballistic particle acceleration as described in, for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); and Lec1 transformation (WO 00/28058). *Also see,* Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longrnan, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize *via Agrobacterium tumefaciens).*

The polynucleotides of the disclosure may be introduced into plants by contacting plants with a virus or viral nucleic acids. Generally, such methods involve incorporating a polynucleotide construct of the disclosure within a viral DNA or RNA molecule. It is recognized that the an AHASL1 protein of the disclosure may be initially synthesized as part of a viral polyprotein, which later may be processed by proteolysis *in vivo* or *in vitro* to produce the desired recombinant protein. Further, it is recognized that promoters of the disclosure also encompass promoters utilized for transcription by viral RNA polymerases. Methods for introducing polynucleotide constructs into plants and expressing a protein encoded therein, involving viral DNA or RNA molecules, are known in the art. See, for example, U.S. Patent Nos. 5,889,191, 5,889,190, 5,866,785, 5,589,367 and 5,316,931.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present disclosure provides transformed seed (also referred to as 'transgenic seed") having a polynucleotide construct of the disclosure, for example, an expression cassette of the disclosure, stably incorporated into their genome.

The present disclosure may be used for transformation of any plant species, including, but not limited to, monocots and dicots. Examples of plant species of interest include, but are not limited to, com or maize *(Zea mays), Brassica* sp. (e.g., *B. napus, B. rapa, B.juncea),* particularly those *Brassica* species useful as sources of seed oil, alfalfa *(Medicago sativa),* rice *(Oryza sativa),* rye *(Secale cereale),* sorghum *(Sorghum bicolor, Sorghum vulgare),* millet (e.g., pearl millet *(Pennisetum glaucum),* proso millet *(Panicum miliaceum),* foxtail millet *(Setaria italica),* finger millet *(Eleusine coracana)),* sunflower *(Helianthus annuus),* safflower *(Carthamus tinctorius),* wheat *(Triticum aestivum, T. Turgidum ssp. durum),* soybean *(Glycine max),* tobacco *(Nicotiana tabacum),* potato *(Solanum tuberosum),* peanuts *(Arachis hypogaea),* cotton *(Gossypium barbadense, Gossypium hirsutum),* sweet potato *(Ipomoea batatus),* cassava *(Manihot esculenta),* coffee *(Coffea* spp.), coconut *(Cocos nucifera),* pineapple *(Ananas comosus),* citrus trees *(Citrus* spp.), cocoa *(Theobroma cacao),* tea *(Camellia sinensis),* banana *(Musa* spp.), avocado *(Persea americana),* fig *(Ficus casica),* guava *(Psidium guajava),* mango *(Mangifera indica),* olive *(Olea europaea),* papaya *(Caricapapaya),* cashew *(Anacardium occidentale),* macadamia *(Macadamia integrifolia),* almond *(Prunus amygdalus),* sugar beets *(Beta vulgaris),* sugarcane *(Saccharum* spp.), oats, barley, vegetables, ornamentals, and conifers. Preferably, plants of the present disclosure are crop plants (for example, sunflower, *Brassica sp.,* cotton, sugar, beet, soybean, peanut, alfalfa, safflower, tobacco, com, rice, wheat, rye, barley triticale, sorghum, millet, etc.).

The herbicide resistant Brassica juncea plants of the invention find use in methods for controlling weeds. Thus, the present invention further provides a method for growing a herbicide-resistant **Brassica juncea** plant of the invention. The method comprises applying an effective amount of an herbicide to the weeds and to the herbicide- resistant Brassica juncea plant, wherein the plant has increased resistance to at least one herbicide, particularly an imidazolinone or sulfonylurean herbicide, when compared to a wild-type plant.

By providing plants having increased resistance to herbicides, particularly imidazolinone and sulfonylurean herbicides, a wide variety of formulations can be employed for protecting plants from weeds, so as to enhance plant growth and reduce competition for nutrients. An herbicide can be used by itself for pre-emergence, post-emergence, pre-planting and at planting control of weeds in areas surrounding the plants described herein or an imidazolinone herbicide formulation can be used that contains other additives. The herbicide can also be used as a seed treatment. That is an effective concentration or an effective amount of the herbicide, or a composition comprising an effective concentration or an effective amount of the herbicide can be applied directly to the seeds prior to or during the sowing of the seeds. Additives found in an imidazolinone or sulfonylurean herbicide formulation or composition include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irrigation, dusting, coating, and the like.

The present disclosure provides methods that involve the use of an AHAS-inhibiting herbicide. In these methods, the AHAS-inhibiting herbicide can be applied by any method known in the art including, but not limited to, seed treatment, soil treatment, and foliar treatment.

The present disclosure provides methods for enhancing the tolerance or resistance of a plant, plant tissue, plant cell, or other host cell to at least one herbicide that interferes with the activity of the AHAS enzyme. Preferably, such an AHAS-inhibiting herbicide is an imidazolinone herbicide, a sulfonylurean herbicide, a triazolopyrimidine herbicide, a pyrimidinyloxybenzoate herbicide, a sulfonylamino-carbonyltriazolinone herbicide, or mixture thereof. More preferably, such an herbicide is an imidazolinone herbicide, a sulfonylurean herbicide, or mixture thereof. For the present invention, the imidazolinone herbicides include, but are not limited to, PURSUIT^{®} (imazethapyr), CADRE^{®} (imazapic), RAPTOR^{®} (imazamox), SCEPTER^{®} (imazaquin), ASSERT^{®} (imazethabenz), ARSENAL^{®} (imazapyr), a derivative of any of the aforementioned herbicides, and a mixture of two or more of the aforementioned herbicides, for example, imazapyr/imazamox (ODYSSEY^{®}). More specifically, the imidazolinone herbicide can be selected from, but is not limited to, 2- (4-isopropyl-4-methyl-5-oxo-2-imidiazolin-2-yl) -nicotinic acid, [2- (4- isopropyl)-4-] [methyl-5-oxo-2-imidazolin-2-yl)-3-quinolinecarboxylic] acid, [5-ethyl-2-(4-isopropyl-]4-methyl-5-oxo-2-imidazolin-2-yl)-nicotinic acid, 2- (4-isopropyl-4-methyl-5-oxo-2- imidazolin-2-yl)-5- (methoxymethyl)-nicotinic acid, [2- (4-isopropyl-4-methyl-5-oxo-2-] imidazolin-2-yl)-5-methylnicotinic acid, and a mixture of methyl [6- (4-isopropyl-4-] methyl-5-oxo-2-imidazolin-2-yl) -m-toluate and methyl [2- (4-isopropyl-4-methyl-5-] oxo-2-imidazolin-2-yl) -p-toluate. The use of 5-ethyl-2- (4-isopropyl-4-methyl-5-oxo- 2-imidazolin-2-yl) -nicotinic acid and [2- (4-isopropyl-4-methyl-5-oxo-2-imidazolin-2-] yl)-5- (methoxymethyl)-nicotinic acid is preferred. The use of [2- (4-isopropyl-4-) methyl-5-oxo-2-imidazolin-2-yl)-5- (methoxymethyl)-nicotinic acid is particularly preferred.

For the present invention, the sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl, halosulfuron, azimsulfuron, cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfuron, a derivative of any of the aforementioned herbicides, and a mixture of two or more of the aforementioned herbicides. The triazolopyrimidine herbicides of the invention include, but are not limited to, cloransulam, diclosulam, florasulam, flumetsulam, metosulam, and penoxsulam. The pyrimidinyloxybenzoate herbicides of the invention include, but are not limited to, bispyribac, pyrithiobac, pyriminobac, pyribenzoxim and pyriftalid. The sulfonylamino-carbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Accordingly, the herbicides of the present invention further include pyrimidinylthiobenzoate herbicides, including, but not limited to, the pyrimidinyloxybenzoate herbicides described above.

Prior to application, the AHAS-inhibiting herbicide can be converted into the customary formulations, for example solutions, emulsions, suspensions, dusts, powders, pastes and granules. The use form depends on the particular intended purpose; in each case, it should ensure a fine and even distribution of the compound according to the disclosure.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents are water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.

Examples of suitable carriers are ground natural minerals (for example kaolins, clays, talc, chalk) and ground synthetic minerals (for example highly disperse silica, silicates).

Suitable emulsifiers are nonionic and anionic emulsifiers (for example polyoxyethylene fatty alcohol ethers, alkylsulfonates and arylsulfonates). Examples of dispersants are lignin-sulfite waste liquors and methylcellulose.

Suitable surfactants used are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents are for example antifoaming agents based on silicon or magnesium stearate. Seed Treatment formulations may additionally comprise binders and optionally colorants.

Binders can be added to improve the adhesion of the active materials on the seeds after treatment. Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol^{®}, Polymin^{®}), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An example of a suitable gelling agent is carrageen (Satiagel^{®}). Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0.01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the disclosure.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS) Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS- inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG)
      Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   I) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds.

In a preferred embodiment a FS formulation is used for seed treatment. Typically, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

The present invention provides non-transgenic seeds of the herbicide- resistant Brassica juncea plants of the present invention. Such seeds include, for example, non- transgenic *Brassica* seeds comprising the herbicide-resistance characteristics of the plant of J04E-0130, or J04E-0122.

For seed treatment, seeds of the herbicide resistant Brassica juncea plants according to the present invention are treated with herbicides, preferably herbicides selected from the group consisting of AHAS-inhibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, rnetsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide.

The term seed treatment comprises all suitable seed treatment techniques known in the art, such as seed dressing, seed coating, seed dusting, seed soaking, and seed pelleting.

In accordance with one variant of the present disclosure, a further subject of the disclosure is a method of treating soil by the application, in particular into the seed drill: either of a granular formulation containing the AHAS-inhibiting herbicide as a composition/formulation, e.g .a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

The present invention also comprises seeds coated with or containing with a seed treatment formulation comprising at least one ALS inhibitor selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid and pyrithiobac.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.1 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

The present disclosure provides a method for combating undesired vegetation or controlling weeds comprising contacting the seeds of the resistant plants according to the present disclosure before sowing and/or after pregermination with an AHAS-inhibiting herbicide. The method can further comprise sowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds of the present disclosure include, for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, and Taraxacum. Monocotyledonous weeds include, but are not limited to, weeds of of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, and Apera.

In addition, the weeds of the present disclosure can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element" means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration.

### Example 1-AHAS in vitro enzyme assay

The AHAS enzyme assay is a quick colourmetric method that is used to quantitate the tolerance levels of different samples by measuring the level of activity of the AHAS enzyme in the presence of AHAS inhibitors, as described by Singh et al. (Anal. Biochem. 171:173-179, 1988). Two types of tests were used: a basic test using only one inhibitor and an intensive test that requires the use of two inhibitors. Both tests indicate levels of imidazolinone tolerance with the intensive test being able to pinpoint slight tolerance level differences evident between some plant lines. AHAS Assay Stock Solution contains: 0.2M of monobasic sodium phosphate + 0.2M of dibasic sodium phosphate + 50 mM 1,1Cyclopropane Dicarboxylic Acid (CPCA) + Full Strength Murashige & Skoogs basal salts + ImM Imazamox (AC 299,263 tech grade ) + 5% H2S04 + 2M NaOH + 2.5% α-napthol+0.25% creatine in 1M Phosphate Buffer pH 6.0.

Final AHAS Assay Solutions include three types of solutions: Solution A contains: 10mM Phosphate buffer + 10% M & S media + 500uM CPCA + 0.5% L-Alanine + 50 mM Pyruvate. Solution B contains: Solution A + 2.5uM Imazamox. Solution C contains: Solution B + 0.2uM Chlorsulfuron.

### Basic AHAS Test: Imazamox Inhibitor:

The test was conducted in 96 well plates. Each 96 well plate contained room for 19-21 samples including controls. Each well contained 100ul of AHAS buffer as described below. In a laminar flow hood, the sterile AHAS buffer was aseptically transferred into two solution basins marked A and B. To the 'B' basin, imazamox was added from a stock solution that equated to a concentration of 2.5uM. 100ul of solution A was transferred to all of the odd numbered rows in each plate, and 100ul of solution B was transferred to all even numbered rows.

### Phase 1: Sampling

Four discs were excised from the bottom of the smallest leaf of ten day old seedlings using a cork borer. Plants were sampled prior to the bolting stage, since another AHAS gene is activated after this growth stage, hence, potentially delivering false results. Following excision, the discs were transferred into the wells of the microtitre plate containing the A and B solutions.

Once the entire microtitre plate was füll, it was incubated under fluorescent lights at room temperature for 14-18 hours. To stop the incubation after this time, the plates were frozen in a -80°C freezer.

### Phase 2: Reaction

The AHAS plates were removed from the -80°C freezer and thawed at room temperature or in a 60°C incubator. Twenty-five microlitre of 5% H₂ S0₄ was added to each well. The acidified plates were incubated at 60°C until all discs were completely brown, about 15 minutes. During this time the napthol solution was prepared and subsequently 150ul of the α-napthol / creatine solution was added to each well. Each plate was incubated at 60°C for 15 minutes. After incubation, the difference in AHAS activity was visually compared between imidazolinone and non-imidazolinone samples. The intensity of the "red" color resulting from the AHAS activity was measured using a Microtitre Plate Reader to deliver the quantity value for the imidazolinone and non-imidazolinone samples.

The absorbance of each well was read at 530nm. At this setting, a value was given that was representative of the intensity of red. This intensity of red translated to the amount of AHAS activity in each well. When the AHAS activity of the imazamox well of a given sample was divided into the AHAS activity of the control, a ratio was given in terms of "percent AHAS activity of control".

### Intensive AHAS Test: Imazamox and Chlorsulfuron Inhibitors

The integration of chlorsulfuron, SU, in the AHAS test is based on the tolerance behavior of the PM I and bR genes. PMI and bR are not tolerant to SU whereas PM2 does show some tolerance to SU. A ratio of the SU activity divided into the imazamox, activity gives a unique value for all four tolerance levels (PM1/PM2, PM2, PM1, WT).

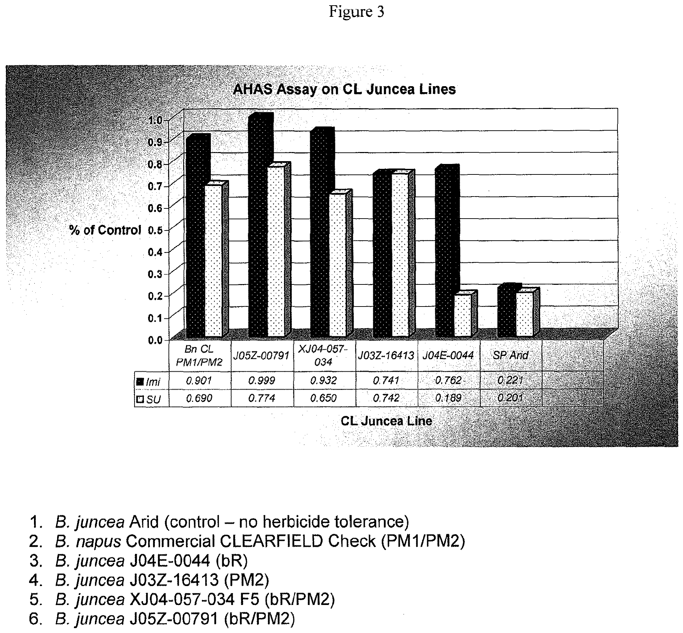

Results are shown in Figure 3 for bR, PM2 and bR/PM2 in *B.juncea* when inhibited with imazamox and chlorsulfuron.

### AHAS Enzyme Activity of Different B.juncea Mutation Combinations in the Presence of Imazamox

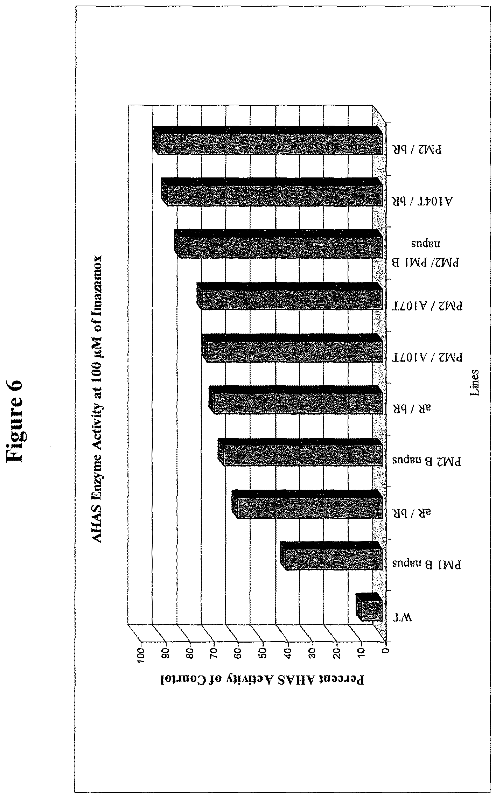

The AHAS enzyme activity in protein extracts from homozygous double haploid (DH) *B.juncea* lines containing different mutation combinations (aR × bR, PM2 × A107T, PM2 × bR, A104T × bR) was measured as a percentage of the activity of the untreated (0 µM imazamox) sample. As a control, protein extracts from three *B. napus* lines were also included: *B. napus* PMI, *B. napus* PM2 and *B. napus* PM1/PM2. The results for these mutant combinations and checks at 100 µM of imazamox are shown in Figure 6.

### Example 2 - HERBICIDE TOLERANCE TESTS IN THE GREENHOUSE

The first experiment was designed to determine if there was a difference in imidazolinone herbicide tolerance between *B.juncea* lines containing one gene (bR or PM2) and two genes (bR/PM2) versus the *B. napus* line containing the two genes (PM1/PM2).

Six individual plants from each line were subjected to each spray treatment.

The imidazolinone herbicide Odyssey^{®} was applied at Ix (17g ai/acre), 2x (34g ai/acre) and 3x (51g ai/acre) at the 2-3 leaf stage. Plants were sprayed at the 2-3 leaf stage approximately 14 days post-planting. The spray chamber was set at 40 psi and the speed was set at '80' (34.98 L / ac). The following calculation was made to make a 25ml stock solution of Odyssey: 17*0.025/34.98=0.1215g of Odyssey granules. This was based on the following value assumptions: the amount of Odyssey required per acre was 17g; and 8.33ml of solution was delivered in each pass of the spray chamber. Merge^{®} was added at a rate of 0.5L/ I00L or 0.000125L or 125uL. After spraying, plants were randomized within the trays. The plants were rated for visual herbicide damage (7-10 days post spray) according to the following rating:
1. plants that do not show any damage
2. plants demonstrating leaf discolouration or slight curling of leaves
3. plants showing major leaf discolouration (e.g. yellowing or purpling) as well as demonstrating some basal branching.
4. plants demonstrating major damage resulting in death or severe set back

Plant height and biomass (plant weight) were measured after the damage was apparent. Comparisons were made between spray treatments and controls for each variety. The results are shown in Table 1.

| **Table 1 : Herbicide injury measurements on *B.juncea* lines containing bR and/or PM2 versus a *B. napus* PM1/PM2 check** | | | | | | | |
|---|---|---|---|---|---|---|---|
| | | | | | | | |

| **N = 6 for all data points** | | | | | | | |
|---|---|---|---|---|---|---|---|
| | | | | | | | |

| | | **Spray** | **Injury** | **Plant height** | | **Plant weight** | |
|---|---|---|---|---|---|---|---|
| **Variety** | | **Rate** | **(1-4)** | **(cm)** | **(% control)** | **(g)** | **(%control)** |
| | | | | | | | |
| **Commercial B. *napus*** | | 0 | 1.0 | 7.3 | 100.0 | 1.120 | 100.0 |
| **(PM1 + PM2)** | | 1 | 1.0 | 6.0 | 81.4 | 1.020 | 91.1 |
| | | 3 | 1.1 | 5.5 | 75.4 | 1.130 | 100.9 |
| | | | | | | | |
| ***B.juncea* J03Z-16413** | | 0 | 1.0 | 8.1 | 100.0 | 1.200 | 100.0 |
| **(PM2)** | | 1 | 1.0 | 7.3 | 90.4 | 1.160 | 96.7 |
| | | 3 | 1.6 | 6.4 | 78.8 | 1.200 | 100.0 |
| | | | | | | | |
| ***B.juncea* JO5Z-00791** | | 0 | 1.0 | 6.1 | 100.0 | 0.690 | 100.0 |
| **(bR + PM2)** | | 1 | 1.0 | 6.2 | 101.0 | 0.750 | 108.7 |
| | | 3 | 1.1 | 5.6 | 91.0 | 0.850 | 123.2 |
| | | | | | | | |
| ***B.juncea* XJ 04-057-034** | | 0 | 1.0 | 7.6 | 100.0 | 1.230 | 100.0 |
| **(bR + PM2)** | | 1 | 1.0 | 7.0 | 92.2 | 1.210 | 98.4 |
| | | 3 | 1.2 | 5.3 | 70.5 | 1.250 | 101.6 |
| | | | | | | | |
| ***B.juncea* J04E-0044** | | 0 | 1.0 | 7.0 | 100.0 | 0.780 | 100.0 |
| **(bR)** 11.46.694.10.75096.2 | | | | | | | |
| | | 32.83.144.30.29037.2 | | | | | |
| | | | | | | | |
| ***B.juncea* Arid** | | 01.07.2100.01.080100.0 | | | | | |
| **(wild type)** | | 13.32.737.50.0908.3 | | | | | |
| | | 33.82.737.10.0403.7 | | | | | |

The second experiment was designed to compare the different mutations, bR, bR/PM2, PM2, aR, Al 04T, and Al 07T in *B.juncea* when treated with different rates of imazamox in the greenhouse. Samples used for the Imazamox Spray Test (Second Greenhouse Experiment) are shown in table 2 below.

**Table 2: B.juncea lines used in the second greenhouse experiment**

| Entry No | Species | Mutatio | Line | Note/Rep |
|---|---|---|---|---|
| 1 | *B juncea* | aR | J04E-0139 | M4 aR S653N A genome |
| 2 | *B juncea* | A107T | J04E-0130 | M3 A122T B genome |
| 3 | *B juncea* | bR | J04E-0044 | M3 bR S653N B genome |
| 4 | *B juncea* | Al04T | J04E-0122 | M3 Al 22T A genome |
| 5 | *B juncea* | - | Arid | Lot C3J3 |
| 6 | *B juncea* | bR/PM | JO5Z-07801 | DH₁ bR/PM2 |
| 7 | *B juncea* | PM2 | J03Z-03315 | PM2 Lot M5T2-002 |

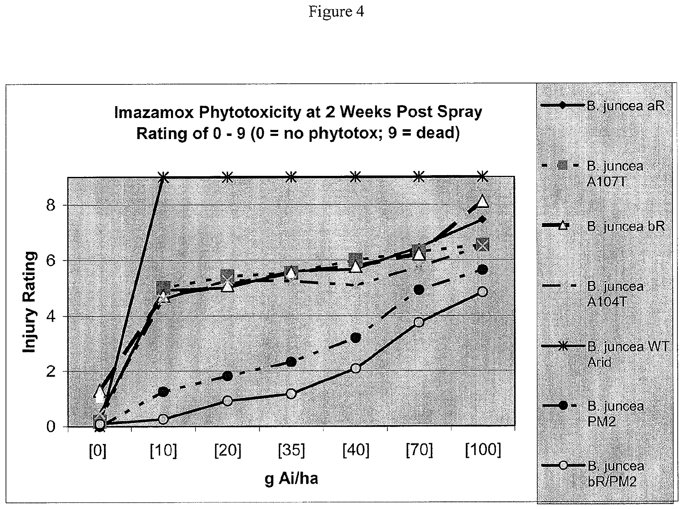

Twelve individual plants per line were subjected to each treatment level, as illustrated in Table 3. There were 7 treatments Imazamox (Raptor^{®}) + 0.5% v/v Merge^{®}. Plants were treated at the 1-2 true leaf stage. The results are shown in Figure 4.

**Table 3. Treatment levels**

| Treatment | Imazamox (g ai/ha) |
|---|---|
| 1 | 0 |
| 2 | 10 |
| 3 | 20 |
| 4 | 35 |
| 5 | 40 |
| 6 | 70 |
| 7 | 100 |

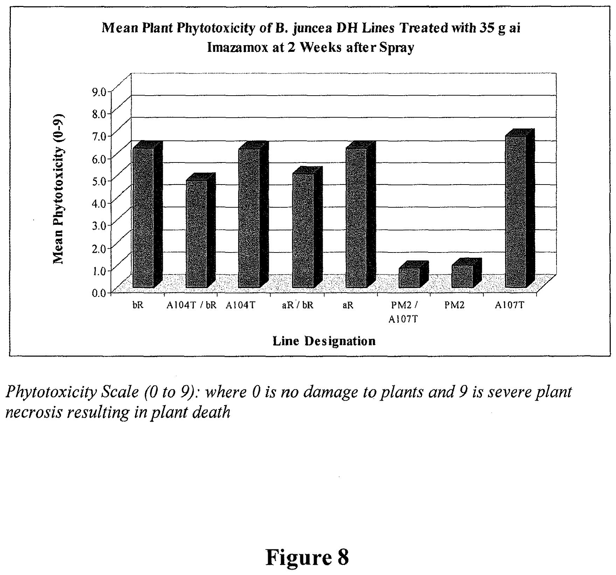

In another greenhouse experiment, *B.juncea* DH lines were produced from crosses between *B.juncea* lines which contained an A genome AHAS mutation (aR, A104T, or PM2) and *B.juncea* lines which contained a B genome AHAS mutation (bR, Al 07T). Those DH lines that were confirmed to be homozygous for both A genome and B genome mutations (ex. aR/bR or A104T/bR, etc.) were selected for subsequent greenhouse herbicide tolerance testing with 0, 35, 70 and 100 g ai/ha of imazamox (Raptor^{®} + 0.75% Merge^{®}). Phytotoxicity was rated on a scale from 0 to 9, where 0 was equivalent to no crop injury and 9 was equivalent to severe plant necrosis leading to plant death. The results of these phytotoxicity curves are shown in Figure 8.

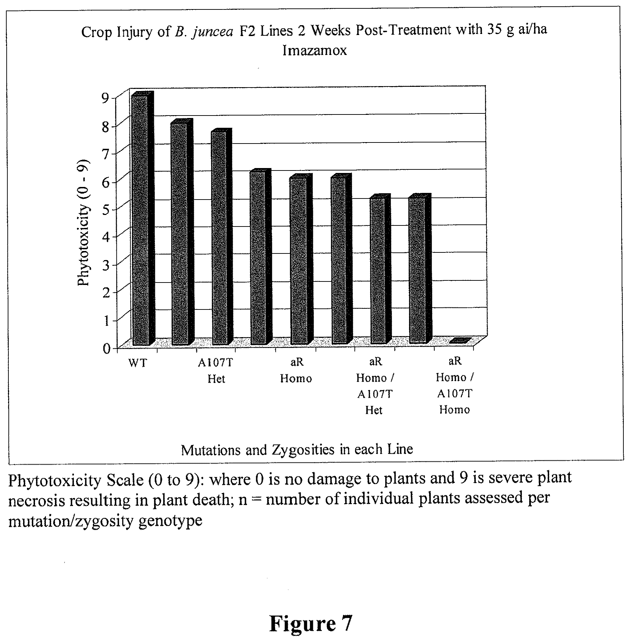

For those combinations where no double homozygous DH line was identified (such was the case for the aR/A 107T mutant combination) a segregating F2 population was planted out in the greenhouse. Each F2 individual was sequenced to determine the nature of the mutation and zygosity, and then sprayed with 35 g ai/ha of imazamox to determine the respective injury phenotype. The results of the genotype to crop injury phenotype relationship for the aR and A 107T mutations are shown in Figure 7.

### Example 3 - HERBICIDE TOLERANCE TESTS IN THE FIELD

Four *B.juncea* entries and one *B. napus* entry were tested in randomized split block design trials (4 repetitions) across four locations in North Dakota for herbicide tolerance (refer to Table 4 for the various treatments) and yield. The plots were a minimum 1.5 × 5 m large and individual plots were swathed and harvested at maturity. Of the four *B.juncea* entries, one entry was a PM1/PM2 *B.juncea* line that was produced by introgressing both the PMI and PM2 mutations from *Brassica napus* into *Brassica juncea* by conventional backcrossing techniques, followed by two generations of selfing to produce homozygous *B.juncea* PM1/PM2. The other three *B.juncea* entries were different genotypes of *B.juncea* containing the B genome bR mutation stacked together with the PM2 mutation. All bR/PM2 B. *juncea* lines were homzoygous for both mutations. The *B. napus* entry was a CLEARFIELD^{®} commercial check variety homozygous for the PM1/PM2 mutations. Crop injury ratings (% phytotoxicity) were taken 5 to 7 days after treatment (DAT) and 18 to 24 DAT. The mean percent phytotoxicity from one of the four locations is presented in Table 5.

**Table 4: Herbicide Treatments**

| | | |
|---|---|---|
| **Treatments:** | | 1. Untreated |
| | | 2. 1x rate of the following CLEARFIELD^{®} canola herbicide products: 30 gai/ha ODYSSEY^{®} +0.5% (v/v) MERGE^{®} |
| **Spray volume:** | 100 liters/ha | |
| **Growth Stage:** | 2-4 leaves | |

| | | |
|---|---|---|
| ^{®}CLEARFIELD and the unique CLEARFIELD symbol are registered trademarks of BASF | | |

**Table 5: The Mean Percent Phytotoxicity and Mean Yield of B.juncea PM1/PM2 and B.juncea bR/PM2 Entries Following a Ix Herbicide Application of Odyssey at one Location in Velva, North Dakota.**

| Entry | Treatment | Mean % Phytotoxicity 5 - 7 DAT | Mean % Phytotoxicity 18 - 24 DAT | Mean Vield KG/HA | |
|---|---|---|---|---|---|
| **B. juncea PM1/PM2** | **Untreated** | **0.00** | **0.00** | **896** | |
| B. napus PM1/PM2 | Untreated | 0.00 | 0.00 | 1119 | |
| B. juncea bR/PM2 (S006) | Untreated | 0.00 | 0.00 | 1217 | |
| B. juncea bR/PM2 (S007) | Untreated | 0.00 | 0.00 | 1382 | |
| B. juncea bR/PM2 (S008) | Untreated | 0.00 | 0.00 | 1343 . | |
| **B. juncea PM1/PM2** | **1×Odyssey** | **48.75** | **50.00** | **351** | |
| B. napus PM1/PM2 | 1x Odyssey | 13.75 | 3.75 | 910 . | |
| B. juncea bR/PM2 (S006) | 1x Odyssey | 3.75 | 1.25 | 1231 . | |
| B. juncea bR/PM2 (S007) | 1x Odyssey | 6.25 | 1.25 | 1334 . | |
| B. juncea bR/PM2 (S008) | 1x Odyssey | 5.00 | 2.50 | 1527 . | |
| | | 5.01 | 3.40 | 254 | **LSD (P=.05)** |
| | | | | 13.96 | **cv** |

The results in Table 5 indicated that the PM1/PM2 introgressed mutations in *B. juncea* do not have adequate tolerance for commercialization. The post-herbicide (Odyssey^{®}) phytotoxicity ratings for the PM1/PM2 *B.juncea* line were in the range of 25 to 50% for all tested locations (Velva, Mohall, Fargo, Hettinger) while the yield on the Odyssey^{®}-treated PM1/PM2 *B.juncea* entry was reduced, on average, by 50% versus the unsprayed PM1/PM2 *B.juncea* entry. The bR/PM2 *B.juncea* entries (S006, S007, S008) demonstrated no phytotoxicity at a Ix Odyssey^{®} treatment for any of the tested locations and did not demonstrate any significant decrease in yield. The bR/PM2 *B.juncea* entries also demonstrated lower phytotoxicity ratings than the PM1/PM2 *B. napus* entry at all locations.

Similarly, twenty-eight different *B.juncea* entries (genotypes) containing the bR/PM2 stacked mutation, along with one *B.juncea* PM2 only entry (J03Z-16413) and one commercial CLEARFIELD^{®} *B. napus* check containing the PM1/PM2 stacked mutations, were field tested at three locations. A randomized complete block design (1 treatment) consisting of 2 replications was used where the average plot size was 1.5 m × 5 m. Regional canola seeding rates were used and individual plot seeding rates were adjusted to the same seeding density for each location, based on each entry's 1000 seed weight. Herbicide was applied to all entries in this trial as shown in table 6 below.

**Table 6: Herbicide treatment**

| | |
|---|---|
| **Treatments:** | **2x rate of the following CLEARFIELD^{®} canola herbicide product:** |
| | 70 g ai/ha BEYOND^{®} +0.5% (v/v) MERGE^{®} |
| **Spray volume:** | 100 liters/ha |
| **Growth Stage:** | 2-4 leaves |
| ^{®} CLEARFIELD and the unique CLEARFIELD symbol are registered trademarks of BASF | |

**Table 7: Agronomic Ratings**

| | |
|---|---|
| KG_HA | Yield in kilograms per hectare, converted from gm/plot using harvested area - 7% moisture basis. |
| % Checks | Relative yield vs. mean of 4 checks. |
| KG_HA | Yield in kilograms per hectare, converted from gm/plot using harvested area - 7% moisture basis. |
| AGRON | Agronomic rating on a 1 to 9 scale, 1 is verv poor, 9 is verv qood. |
| INJURY | Visual % injury done at an early stage - 7 to 10 days after spraying with Bevond^{®}. |
| FLOWER DAYS | Days from seeding to first flower. |
| FLOWER DURATION | Days from first flower to end of flower. |
| MATURITY | Days from seeding to maturity. |
| HGT | Height at maturity in cm. |
| LODGE | Lodging rated on a 1 to 5 scale, 1 is no lodging, 5 is significant lodging |

**Table 8: Field test results**

| **Imidazolinone tolerant *B.juncea* Preliminary Field Trial** | | | | | | | | | | | | | |
|---|---|---|---|---|---|---|---|---|---|---|---|---|---|
| **Summary of 3 locations (Canada, Year 1), 2 reps per location, all plots sprayed with 2x Beyond** | | | | | | | | | | | | | |
| **Check = Entry2 (Commercial CLEARFIELD^{®}, CL, *B.napus* line: PM1/PM2)** | | | | | | | | | | | | | |
| | | | | | Herbicide | | | | | | | | |
| | | | | Aqron | Injury | Flower | | Maturity | Hqt | Lodge | Yield | | |
| Name | Mutation Event | Species | Entry | (1-9) | (%) | (days) | (duration) | (days) | (cm) | (1-5) | (kg/ha) | % of Check: Entry 2) | |
| | | | | | | | | | | | | | |
| J03Z-16413 | PM2 | B. juncea | 1 | 3.6 | 19.2 | 48.7 | 20.0 | 95.5 | 127.7 | 2.2 | 2661.8 | 78.2 | |
| Commercial CL *B*. *napus* | PM1/PM2 | B. napus | 2 | 6.8 | 12.3 | 47.2 | 14.9 | 91.5 | 120.9 | 1.8 | 3402.9 | 100.0 | |
| J05Z-08310 | bR/PM2 | B. juncea | 3 | 5.6 | 2.7 | 45.3 | 17.1 | 91.8 | 142.6 | 1.2 | 3468.4 | 101.9 | |
| J05Z-08333 | bR/PM2 | B. juncea | 4 | 5.3 | 2.0 | 46.2 | 16.8 | 92.1 | 142.7 | 1.5 | 3696.9 | 108.6 | |
| J05Z-08347 | bR/PM2 | B. juncea | 5 | 6.0 | 2.0 | 45.0 | 17.7 | 90.0 | 137.6 | 1.6 | 3685.3 | 108.3 | |
| J05Z-08433 | bR/PM2 | B. juncea | 6 | 6.3 | 1.7 | 44.2 | 18.1 | 89.3 | 139.8 | 1.3 | 3555.9 | 104.5 | |
| J05Z-07317 | bR/PM2 | B. juncea | 7 | 5.2 | 2.7 | 45.3 | 17.1 | 92.3 | 147.9 | 1.4 | 3492.4 | 102.6 | |
| J05Z-07322 | bR/PM2 | B. juncea | 8 | 5.0 | 3.3 | 44.0 | 17.5 | 88.9 | 143.4 | 1.8 | 3248.3 | 95.5 | |
| J05Z-07366 | bR/PM2 | B. juncea | 9 | 5.7 | 2.3 | 43.4 | 18.5 | 91.3 | 150.6 | 1.5 | 4082.3 | 120.0 | |
| J05Z-09273 | bR/PM2 | B. juncea | 10 | 6.5 | 2.7 | 44.2 | 15.2 | 89.7 | 148.2 | 1.5 | 3754.5 | 110.3 | |
| J05Z-06609 | bR/PM2 | B. juncea | 11 | 5.7 | 2.3 | 43.1 | 17.3 | 90.4 | 146.4 | 1.6 | 4315.7 | 126.8 | |
| J05Z-07756 | bR/PM2 | B. juncea | 12 | 6.1 | 7.7 | 44.2 | 17.7 | 89.5 | 136.6 | 1.2 | 3537.0 | 103.9 | |
| J05Z-07814 | bR/PM2 | B. juncea | 13 | 6.1 | 8.7 | 42.0 | 18.2 | 87.7 | 137.0 | 1.7 | 3758.0 | 110.4 | |
| J05Z-07830 | bR/PM2 | B. juncea | 14 | 6.5 | 2.8 | 44.8 | 18.2 | 90.6 | 143.7 | 1.1 | 3642.5 | 107.0 | |
| J05Z-07848 | bR/PM2 | B. juncea | 15 | 5.7 | 7.3 | 43.0 | 18.8 | 88.3 | 133.7 | 1.4 | 3570.0 | 104.9 | |
| J05Z-07937 | bR/PM2 | B. juncea | 16 | 6.2 | 5.0 | 43.2 | 17.2 | 89.7 | 132.7 | 1.6 | 3570.6 | 104.9 | |
| J05Z-07952 | bR/PM2 | B. juncea | 17 | 6.8 | 4.7 | 44.6 | 16.9 | 91.8 | 136.3 | 1.4 | 3545.1 | 104.2 | |
| J05Z-07957 | bR/PM2 | B. juncea | 18 | 5.9 | 2.0 | 44.4 | 18.3 | 89.9 | 139.6 | 1.2 | 3839.5 | 112.8 | |
| J05Z-07975 | bR/PM2 | B. juncea | 19 | 5.3 | 3.0 | 41.8 | 16.9 | 89.9 | 128.6 | 2.0 | 3642.1 | 107.0 | |
| J05Z-07984 | bR/PM2 | B. juncea | 20 | 5.4 | 9.5 | 43.9 | 17.8 | 90.2 | 130.1 | 1.5 | 3637.3 | | 106.9 |
| J05Z-07989 | bR/PM2 | B. juncea | 21 | 7.3 | 2.3 | 44.3 | 18.0 | 89.9 | 134.0 | 1.2 | 3989.5 | | 117.2 |
| J05Z-07994 | bR/PM2 | B.juncea | 22 | 6.7 | 6.0 | 43.2 | 18.6 | 89.3 | 129.7 | 1.4 | 3710.6 | | 109.0 |
| J05Z-08018 | bR/PM2 | B. juncea | 23 | 7.1 | 1.7 | 43.3 | 18.6 | 88.8 | 129.2 | 1.4 | 3977.1 | | 116.9 |
| J05Z-08029 | bR/PM2 | B. juncea | 24 | 6.5 | 2.0 | 45.0 | 18.3 | 92.2 | 143.1 | 1.1 | 3469.5 | | 102.0 |
| J05Z-08045 | bR/PM2 | B. juncea | 25 | 5.9 | 2.3 | 43.0 | 18.2 | 92.1 | 131.2 | 1.5 | 3304.2 | | 97.1 |
| J05Z-08122 | bR/PM2 | B. juncea | 26 | 6.8 | 2.3 | 43.4 | 16.0 | 87.9 | 131.6 | 1.2 | 3760.7 | | 110.5 |
| J05Z-08131 | bR/PM2 | B. juncea | 27 | 5.2 | 4.7 | 42.3 | 15.5 | 88.1 | 129.7 | 2.0 | 3411.9 | | 100.3 |
| J05Z-08133 | bR/PM2 | B. iuncea | 28 | 7.2 | 6.0 | 42.3 | 17.2 | 91.4 | 134.9 | 1.1 | 3947.6 | | 116.0 |
| J05Z-08159 | bR/PM2 | B. juncea | 29 | 6.8 | 4.7 | 43.2 | 17.6 | 88.4 | 135.1 | 1.3 | 3959.5 | | 116.4 |
| J05Z-08190 | bR/PM2 | B. juncea | 30 | 7.1 | 4.3 | 43.5 | 16.1 | 88.6 | 123.3 | 1.2 | 3883.3 | | 114.1 |
| Grand Mean | | | | 6.0 | 4.7 | 44.1 | 17.5 | 90.2 | 136.2 | 1.4 | 3650.7 | | |
| CV | | | | 12.5 | 58.7 | 0.8 | 5.6 | 2.3 | 3.0 | 23.0 | 8.1 | | |
| LSD | | | | 1.3 | 3.7 | 0.6 | 1.7 | 2.8 | 6.9 | 0.6 | 401.9 | | |

Two additional, different *B.juncea* entries (genotypes) containing the bR/PM2 stacked mutation and one commercial **CLEARFIELD^{®}** *B. napus* check containing the PM1/PM2 stacked mutations, were field tested at multiple locations over two successive years. Regional canola seeding rates were used and individual plot seeding rates were adjusted to the same seeding density for each location, based on each entry's 1000 seed weight. Herbicide (Beyond^{®}) was applied to all entries in this trial as shown in Table 9 below.

**Table 9:**

| | | | | | Injury | | Flower | Maturity | Height | Yield |
|---|---|---|---|---|---|---|---|---|---|---|
| Location | Variety | | | Rate | 7-10 day | 14-21 day | (days) | (days) | (cm) | (kg/ha) |
| | | Year 1 | | | | | | | | |
| Watrous | | B. napus check | PM1/PM2 | | 0.0 | 0.0 | 46 | 89.8 | 134.5 | 3867.1 |
| Watrous | | B. napus check | PM1/PM2 | 1x | 8.3 | 0.0 | 46.3 | 87.8 | 132.5 | 4463.8* |
| Watrous | | B. napus check | PM1/PM2 | 2x | 12.5 | | | | | |
| Watrous | | J05Z-08376 | bR/PM2 | | 0.0 | 0.0 | 44.8 | 94.8 | 158.8 | 4661.7 |
| Watrous | | J05Z-08376 | bR/PM2 | 1x | 0.0 | 0.0 | 45 | 93 | 156.8 | 4807.8 |
| Watrous | | J05Z-08376 | bR/PM2 | 2x | 3.0 | | | | | |
| Watrous | | J05Z-07784 | bR/PM2 | | 0.0 | 0.0 | 43.8 | 94.3 | 151.3 | 4571.6 |
| Watrous | | J05Z-07784 | bR/PM2 | 1x | 0.5 | 0.0 | 44.3 | 95.3 | 157.5 | 4870.7 |
| Watrous | | J05Z-07784 | bR/PM2 | 2x | 2.0 | | | | | |
| | | CV(%) | | | | | | | | 7.4 |
| | | LSD (0.05) | | | 1.6 | 1.4 | 0.64 | 3.12 | 10.1 | 450.6 |
| | | | | | | | | | | |
| Avonlea | | B. napus check | PM1/PM2 | | 0.0 | 0.0 | 50.5 | 90.8 | 117.8 | 2940.7 |
| Avonlea | | B. napus check | PM1/PM2 | 1x | 2.5 | 0.0 | 50.5 | 90.5 | 115.8 | 3150.7 |
| Avonlea | | B. napus check | PM1/PM2 | 2x | 7.0 | | | | | |
| Avonlea | | J05Z-08376 | bR/PM2 | | 0.0 | 0.0 | 48.5 | 89.3 | 138.8 | 3295.4 |
| Avonlea | | J05Z-08376 | bR/PM2 | 1x | 1.5 | 0.5 | 48.3 | 90.3* | 140.8 | 3608.4* |
| Avonlea | | J05Z-08376 | bR/PM2 | 2x | 1.0 | | | | | |
| Avonlea | | J05Z-07784 | bR/PM2 | | 0.0 | 0.0 | 46.5 | 88.8 | 131.3 | 3532.7 |
| Avonlea | | J05Z-07784 | bR/PM2 | 1x | 2.0 | 0.0 | 46.5 | 89.5 | 138.8* | 3438.5 |
| Avonlea | | J05Z-07784 | bR/PM2 | 2x | 1.0 | | | | | |
| | | CV(%) | | | | | | | | 6.6 |
| | | LSD (0.05) | | | 2.7 | 1.8 | 0.76 | 0.85 | 6.9 | 309.7 |
| | | | | | | | | | | |
| Hanley | | B. napus check | PM1/PM2 | | 0.0 | 0.0 | 49.8 | 92.8 | 124.5 | 2473.6 |
| Hanley | | B. napus check | PM1/PM2 | 1x | 15.0 | 9.3 | 49.3 | 92.0 | 123.8 | 3057.4* |
| Hanley | | B. napus check | PM1/PM2 | 2x | 20.0 | | | | | |
| Hanley | | J05Z-08376 | bR/PM2 | | 0.0 | 0.0 | 48.8 | 86.0 | 147.3 | 2915.1 |
| Hanley | | J05Z-08376 | bR/PM2 | 1x | 2.8 | 11.0 | 48.0 | 84.3 | 146.3 | 3316.9* |
| Hanley | | J05Z-08376 | bR/PM2 | 2x | 2.0 | | | | | |
| Hanley | | J05Z-07784 | bR/PM2 | | 0.0 | 0.0 | 48.3 | 87.8 | 142.3 | 3356.3 |
| Hanley | | J05Z-07784 | bR/PM2 | 1x | 2.8 | 2.0 | 47.0 | 87.3 | 141.8 | 3702.7* |
| Hanley | | J05Z-07784 | bR/PM2 | 2x | 3.5 | | | | | |
| | | CV (%) | | | | | | | | 6.3 |
| | | LSD (0.05) | | | 4.7 | 3.6 | 3.2 | 2.52 | 7.4 | 277 |
| | | | | | | | | | | |
| Craik | | B. napus check | PM1/PM2 | | 0.0 | 0.0 | 49.0 | 86.0 | 116.5 | 3394.6 |
| Craik | | B. napus check | PM1/PM2 | 1x | 2.0 | 0.0 | 49.0 | 86.0 | 110.5 | 3213.5 |
| Craik | | J05Z-08376 | bR/PM2 | | 0.0 | 0.0 | 47.0 | 87.8 | 132.0 | 3137.4 |
| Craik | | J05Z-08376 | bR/PM2 | 1x | 0.8 | 0.3 | 46.5 | 85.0 | 121.8* | 2904.6 |
| Craik | | J05Z-07784 | bR/PM2 | | 0.0 | 0.0 | 46.0 | 88.8 | 132.8 | 3633.9 |
| Craik | | J05Z-07784 | bR/PM2 | 1x | 3.5 | 0.0 | 46.3 | 87.0 | 130.0 | 3626.0 |
| | | CV (%) | | | | | | | | 8.3 |
| | | LSD (0.05) | | | 5.0 | 2.1 | 1.0 | 3.8 | 7.4 | 365.5 |
| | | | | | | | | | | |
| Trochu | | B. napus check | PM1/PM2 | | 0.0 | 0.0 | 49.8 | 102.0 | 132.5 | 4791.1 |
| Trochu | | B. napus check | PM1/PM2 | 1x | 0.5 | 4.3 | 49.8 | 102.0 | 136.3 | 4831.1 |
| Trochu | | J05Z-08376 | bR/PM2 | | 0.0 | 0.0 | 47.8 | 103.3 | 148.8 | 6021.3 |
| Trochu | | J05Z-08376 | bR/PM2 | 1x | 1.0 | 0.5 | 47.8 | 101.3* | 150.0 | 5954.2 |
| Trochu | | J05Z-07784 | bR/PM2 | | 0.0 | 0.0 | 47.0 | 103.5 | 148.8 | 6413.1 |
| Trochu | | J05Z-07784 | bR/PM2 | 1x | 3.0 | 0.5 | 45.8* | 103.5 | 153.0 | 5954.2 |
| | | CV (%) | | | | | | | | 8.3 |
| | LSD (0.05) | | | | 13.1 | 14.5 | 0.9 | 1.9 | 10.7 | 627.9 |
| | | | | | | | | | | |

| | Year 2 | | | | | | | | | |
|---|---|---|---|---|---|---|---|---|---|---|
| Watrous | B. napus check | | PM1/PM2 | | 0.0 | 0.0 | 36.8 | 87.8 | 122.0 | 3886.1 |
| Watrous | B. napus check | | PM1/PM2 | 2x | 0.0 | 1.0 | 37.0 | 85.5 | 129.3 | 3506.9 |
| Watrous | J05Z-08376 | | bR/PM2 | | 0.0 | 0.0 | 35.8 | 81.8 | 130.3 | 3525.9 |
| Watrous | J05Z-08376 | | bR/PM2 | 2x | 2.5 | 0.0 | 35.8 | 82.8 | 136.8 | 3615.5 |
| Watrous | J05Z-07784 | | bR/PM2 | | 0.0 | 0.0 | 35.3 | 86.0 | 131.3 | 3952.8 |
| Watrous | J05Z-07784 | | bR/PM2 | 2x | 2.0 | 1.0 | 35.8 | 84.5 | 127.8 | 3818.0 |
| IWatrous | PM2 | | PM2 | | 0.0 | 0.0 | 39.8 | >90 | 118.0 | 3987.0 |
| IWatrous | PM2 | | PM2 | 2x | 22.5 | 25.0 | 44.3* | >90 | 103.5* | 2984.9* |
| | CV (o/o) | | | | | | | | | 11.7 |
| | LSD (0.05) | | | | 5.7 | 5.8 | 0.9 | 2.9 | 6.6 | 584.4 |
| | | | | | | | | | | |
| Craik | 8. napus check | | PM1/PM2 | | 0.0 | 0.0 | 49.5 | 84.3 | 114.5 | 2165.0 |
| Craik | 8. napus check | | PM1/PM2 | 2x | 2.5 | 0.3 | 49.3 | 84.5 | 115.0 | 2171.3 |
| Craik | J05Z-08376 | | bR/PM2 | | 0.0 | 0.0 | 47.5 | 87.8 | 139.0 | 2372.6 |
| Craik | J05Z-08376 | | bR/PM2 | 2x | 1.0 | 0.0 | 48.0 | 90.0 | 140.3 | 3080.5* |
| Craik | J05Z-07784 | | bR/PM2 | | 0.0 | 0.0 | 47.8 | 88.5 | 136.8 | 2194.3 |
| Craik | J05Z-07784 | | bR/PM2 | 2x | 1.5 | 0.3 | 47.5 | 88.3 | 133.3 | 3085.5* |
| Craik | PM2 | | PM2 | | 0.0 | 0.0 | 52.0 | 91.0 | 115.0 | 1874.2 |
| Craik | PM2 | | PM2 | 2x | 4.5 | 3.3 | 55.8* | 90.3 | 108.0 | 1980.8 |
| | CV (o/o) | | | | | | | | | 9.9 |
| | LSD (0.05) | | | | 1.8 | 1.0 | 1.1 | 2.4 | 7.9 | 330.1 |
| | | | | | | | | | | |
| Eyebrow | 8. napus check | | PM1/PM2 | | 0.0 | 0.0 | 41.5 | 74.3 | 121.3 | 1687.1 |
| Eyebrow | 8. napus check | | PM1/PM2 | 2x | 1.5 | 0.0 | 42.0 | 72.8 | 114.8 | 1477.1 |
| Eyebrow | J05Z-08376 | | bR/PM2 | | 0.0 | 0.0 | 38.8 | 73.5 | 147.8 | 2034.1 |
| Eyebrow | J05Z-08376 | | bR/PM2 | 2x | 2.8 | 1.0 | 38.0 | 71.8 | 133.8* | 2131.2 |
| Eyebrow | J05Z-07784 | | bR/PM2 | | 0.0 | 0.0 | 39.0 | 72.3 | 134.8 | 1909.5 |
| Eyebrow | J05Z-07784 | | bR/PM2 | 2x | 3.3 | 1.8 | 39.3 | 74.8 | 124.8* | 1903.7 |
| Eyebrow | PM2 | | PM2 | | 0.0 | 0.0 | 44.0 | 78.7 | 113.5 | 1434.4 |
| Eyebrow | PM2 | | PM2 | 2x | 1.9 | 2.8 | 49.5* | 82.3* | 97.0* | 742.4* |
| | CV (o/o) | | | | | | | | | 9.7 |
| | LSD (0.05) | | | | 1.5 | 1.1 | 1.0 | 2.9 | 7.8 | 277.7 |
| | | | | | | | | | | |
| Vulcan | B. napus check | PM1/PM2 | | 0.0 | 0.0 | | | 88.0 | 103.8 | 3527.5 |
| Vulcan | B. napus check | PM1/PM2 | 2x | 1.0 | 0.5 | | | 88.0 | 101.9 | 3202.4 |
| Vulcan | J05Z-08376 | bR/PM2 | | 0.0 | 0.0 | | | 89.8 | 123.8 | 4177.0 |
| Vulcan | J05Z-08376 | bR/PM2 | 2x | 6.3 | 2.8 | | | 85.0 | 118.8 | 3807.9 |
| Vulcan | J05Z-07784 | bR/PM2 | | 0.0 | 0.0 | | | 89.8 | 115.0 | 2995.2 |
| Vulcan | J05Z-07784 | bR/PM2 | 2x | 13.8 | 11.3 | | | 88.5 | 108.8 | 2672.5 |
| Vulcan | PM2 | PM2 | | 0.0 | 0.0 | | | 88.0 | 108.8 | 2566.2 |
| Vulcan | PM2 | PM2 | 2x | 42.5 | 26.3 | | | 91.5 | 100.6 | 2357.2 |
| | CV (%) | | | | | | | | | 13.3 |
| | LSD (0.05) | | | 12.0 | 9.6 | | | 5.2 | 16.4 | 619.6 |
| | | | | | | | | | | |
| Orkney | B. napus check | PM1/PM2 | | 0.0 | 0.0 | 46.3 | | 88.8 | 87.5 | 1407.3 |
| Orkney | B. napus check | PM1/PM2 | 2x | 3.0 | 0.5 | 47.5 | | 90.0 | 77.8* | 1398.0 |
| Orkney | J05Z-08376 | bR/PM2 | | 0.0 | 0.0 | 46.8 | | 88.5 | 89.0 | 2028.8 |
| Orkney | J05Z-08376 | bR/PM2 | 2x | 9.3 | 5.0 | 47.3 | | 92.0* | 93.3 | 2000.8 |
| Orkney | J05Z-07784 | bR/PM2 | | 0.0 | 0.0 | 47.3 | | 91.3 | 91.3 | 2002.3 |
| Orkney | J05Z-07784 | bR/PM2 | 2x | 13.8 | 6:8 | 48.3 | | 91.5 | 93.3 | 2062.8 |
| Orkney | PM2 | PM2 | | 0.0 | 0.0 | 49.0 | | 90.3 | 80.3 | 1315.1 |
| Orkney | PM2 | PM2 | 2x | 10.0 | 11.3 | 51.5* | | 99.5* | 73.5 | 316.4* |
| | CV (%) | | | | | | | | | 10.5 |
| | LSD (0.05) | | | 29.2 | 12.7 | 2.2 | | 3.5 | 9.0 | 256.4 |
| | | | | | | | | | | |
| Hanley | B. napus check | PM1/PM2 | | 0.0 | 0.0 | | | 85.3 | 133.0 | 1084.6 |
| Hanley | B. napus check | PM1/PM2 | 5x | 16.5 | 14.8 | | | 89.5* | 117.5* | 1287.3 |
| Hanley | J05Z-08376 | bR/PM2 | | 0.0 | 0.0 | | | 90.3 | 144.0 | 1808.8 |
| Hanley | J05Z-08376 | bR/PM2 | 5x | 41.5 | 21.5 | | | 88.5 | 143.0 | 1866.3 |
| Hanley | J05Z-07784 | bR/PM2 | | 0.0 | 0.0 | | | 90.8 | 134.0 | 1909.0 |
| Hanley | J05Z-07784 | bR/PM2 | 5x | 24.3 | 14.3 | | | 91.0 | 129.3 | 1761.1 |
| Hanley | PM2 | PM2 | | 0.0 | 0.0 | | | 94.0 | 122.3 | 1249.6 |
| Hanley | PM2 | PM2 | 5x | 26.5 | 74.5 | | | 99.5* | 92.5* | 746.2* |
| | CV (%) | | | | | | | | | 16.0 |
| | LSD (0.05) | | | 21.3 | 14.9 | | | 4.2 | 7.4 | 404.7 |
| | | | | | | | | | | |

Field phytotoxicity data was also obtained from 3 different lines containing both the bR and PM2 mutations (homozygous for bR/PM2), and compared to the field phytotoxicity of a commercial *B. napus* line containing both the PMI and PM2 mutations (homozygous for PM1/PM2). These lines were sprayed with Ix BEYOND^{®} (35 g ai/ha of imazamox) and scored for phytotoxicity at 7 to 10 days after treatment (DAT). *A B.juncea* wild-type line (i.e. not having any AHAS mutations) was also sprayed with IX BEYOND^{®} as a control. The results are provided in Table 10 below.

**Table 10:**

| **Year1 Field Season-Agronomie Performance of *B.junce*asprayed with Ix BEYOND^{®}** | |
|---|---|
| | Percent Phytotoxicity at 7-10 DAT |
| *B. napus* | 5.7 |
| PM1/PM2 | |
| *B.juncea* | 1.2 |
| bR/PM2 | |
| s 002 | |
| *B.juncea* | 2.4 |
| bR/PM2 | |
| s 003 | |
| *B*. *juncea* | 2.8 |
| bR/PM2 | |
| s006 | |

A similar set of experiments was conducted on four additional mid-oleic *B.juncea* lines containing both the bR and PM2 mutations (homozygous bR/PM2) sprayed with 2x BEYOND^{™}. The results are presented in Table 11 below.

**Table 11:**

| **Year 1 Field Season -Agronomie Performance of *B.juncea* lines sprayed with 2xBEYOND^{™}** | |
|---|---|
| | Percent Phytotoxicity at 7-10 DAT |
| *B. napus* | 13.2 |
| PM1/PM2 | |
| *B.juncea* | 4.2 |
| bR/PM2 | |
| J05Z-5105 | |
| *B.juncea* | 1.3 |
| bR/PM2 | |
| J05Z-7146 | |
| *B.juncea* | 2.2 |
| bR/PM2 | |
| J05Z07154 | |
| *B.juncea* | 2.3 |
| bR/PM2 | |
| IJ05Z07160 | |

To study the effect of stacking the bR and PM2 mutations together versus the respective individual mutations, herbicide tolerance field tests were performed on B. juncea entries containing either the PM2 mutation alone, the bR mutation alone, or entries containing both the bR and PM2 mutations. All mutations were homozyous in all entries. A randomized complete block design (4 treatments) consisting of 3 replications was used with an average plot size of 1.5 m × 5 m. Regional canola seeding rates were used and individual plot seeding rates were adjusted to the same seeding density for each location, based on each entry's 1000 seed weight. Herbicide (Beyond^{®}, where a Ix rate was 35 g ai/ha) was applied to all entries in this trial as shown in table 10 below. B.juncea lines containing the single or combined traits were treated with Ox, Ix, 2x, or 4x levels of herbicide and rated at 10 days, 12 days, 14 days, or 28 days after treatment (DAT). Two different people scored phytotoxicity at 10, 12, 14, and/or 28 days after treatment with the respective amounts of herbicide (as shown in Table 10: Scorer 1 versus Scorer 2). The results are presented in Table 12 below.

**Table 12:**

| **Mean Percentage Phytotoxicity (3 Reps)** | | | | |
|---|---|---|---|---|
| | Scorer 1 | Scorer 2 | Scorer 1 | Scorer 1 |
| Entry and Herbicide Rate | 10 DAT | 12 DAT | 14 DAT | 28 DAT |
| bR/PM2 0x | 0.0 | 0.0 | 0.0 | 0.0 |
| bR/PM2 Ix | 4.0 | 7.5 | 2.3 | 0.7 |
| bR/PM2 2x | 11.7 | 10.8 | 10.0 | 3.0 |
| bR/PM2 4x | 25.0 | 21.7 | 28.3 | 15.0 |
| PM2 0x | 0.0 | 0.0 | 0.0 | 0.7 |
| PM2 Ix | 11.7 | 17.5 | 6.7 | 5.7 |
| PM2 2x | 35.0 | 28.3 | 35.0 | 33.3 |
| PM2 4x | 53.3 | 36.7 | 56.7 | 65.0 |
| bR 0x | 0.0 | 0.0 | 0.0 | 0.0 |
| bR Ix | 98.7 | 81.7 | 99.3 | 99.0 |
| bR 2x | 100.0 | 85.0 | 99.7 | 99.3 |
| bR 4x | 100.0 | 93.3 | 100.0 | 73.3 |
| | | | | |
| CV | 16.4 | 9.5 | 19.0 | 48.3 |
| LSD | 10.2 | 5.1 | 11.8 | 26.9 |

To more clearly demonstrate that the tolerance in the bR/PM2 stacked mutant line was greater than the sum of the individual mutant line tolerances (Crop injury or phytotoxocity is shown in Table 12), the actual percent herbicide tolerance of the bR/PM2 stack was compared to the sum of the percent herbicide tolerances of the single bR mutant line plus the single PM2 mutant line (predicted herbicide tolerance) (Table 13). At herbicide levels which challenge or overwhelm the single mutations (most notably at 2x and 4x rates), the level of herbicide tolerance observed in the bR/PM2 stacked mutant line exceeds the herbicide tolerance observed when adding the two individual bR + PM2 tolerances together. The bR/PM2 stacked mutant line exhibits a synergistic level of herbicide tolerance rather than an additive level. This enhanced (synergistic) level of imidazolinone tolerance has been observed in more than 30 different genotypes of *B.juncea* containing the bR/PM2 stacked mutations.

**Table 13:**

| **Percent Imidazolinone Tolerance** | | | | |
|---|---|---|---|---|
| Entry Description | 10 DAT | 12 DAT | 14DAT | 28 DAT |
| bR/PM2 0x | 100.0 | 100.0 | 100.0 | 100.0 |
| bR/PM2 Ix | 96.0 | 92.5 | 97.7 | 99.3 |
| bR/PM2 2x | 88.3 | 89.2 | 90.0 | 97.0 |
| bR/PM2 4x | 75.0 | 78.3 | 71.7 | 85.0 |
| PM2 0x | 100.0 | 100.0 | 100.0 | 99.3 |
| PM2 Ix | 88.3 | 82.5 | 93.3 | 94.3 |
| PM22x | 65.0 | 71.7 | 65.0 | 66.7 |
| PM2 4x | 46.7 | 63.3 | 43.3 | 35.0 |
| bR 0x | 100.0 | 100.0 | 100.0 | 100.0 |
| bR Ix | 1.3 | 18.3 | 0.7 | 1.0 |
| bR 2x | 0.0 | 15.0 | 0.3 | 0.7 |
| bR 4x | 0.0 | 6.7 | 0.0 | 26.7 |

| Predicted Imidazolinone Tolerance based on individual traits | | | | |
|---|---|---|---|---|
| PM2 + bR Ix | 89.6 | 100.8 | 94.0 | 95.3 |
| PM2 + bR 2x | 65.0 | 86.7 | 65.3 | 67.4 |
| PM2 + bR 4x | 46.7 | 70.0 | 43.3 | 61.7 |

In summary, the synergistic or enhanced level of tolerance in *B.juncea* bR/PM2 lines has been shown to be greater than the level of tolerance observed in the *B. napus* PM1/PM2 stacked mutant lines and also much greater than the tolerance observed in the *B.juncea* PM1/PM2 stacked mutant lines (Table 5). The *B.juncea* bR/PM2 lines did not demonstrate any yield penalties when treated with imidazolinone herbicides, while the *B.juncea* PM1/PM2 line demonstrated significant yield penalties when treated with commercial rates of imidazolinone herbicide.

All publications and patent applications mentioned in the specification are indicative of the level of those skilled in the art to which this invention pertains.

The disclosure further includes the following subject matter of WO2009/031031 from which this application is derived.

Disclosed is a *Brassica* plant comprising in its genome at least one copy of an acetohydroxyacid synthase large subunit (AHASL) polynucleotide that encodes a herbicide resistant AHASL polypeptide, wherein the AHASL polypeptide is selected from the group consisting of:
a) a polypeptide having an asparagine at a position corresponding to position 653 of SEQ ID NO:I, or position 638 of SEQ ID NO:2, or position 635 of SEQ ID NO:3;
b) a polypeptide having a threonine at a position corresponding to position 122 of SEQ ID NO:I, or position 107 of SEQ ID NO:4, or position 104 of SEQ ID NO:5; and
c) a polypeptide having a leucine at a position corresponding to position 574 of SEQ ID NO:I, or position 556 of SEQ ID NO:6, of WO2009/031031.

The disclosed plant can be selected from the group consisting of *B.juncea, B. napus, B. rapa, B. carinata, B. oleracea,* and *B. nigra.*

The disclosed plant can comprise at least two copies of an acetohydroxyacid synthase large subunit (AHASL) polynucleotide that encodes a herbicide-resistant AHASL polypeptide, wherein the AHASL polypeptide is selected from the group consisting of:
a) a polypeptide having an asparagine at a position corresponding to position 653 of SEQ ID NO: 1, or position 638 of SEQ ID NO:2, or position 635 of SEQ ID NO:3;
b) a polypeptide having a threonine at a position corresponding to position 122 of SEQ ID NO:I , or position 107 of SEQ ID NO:4, or position 104 of SEQ ID NO: 5; and
c) a polypeptide having a leucine at a position corresponding to position 574 of SEQ ID NO: 1, or position 556 of SEQ ID NO:6.

## Claims

1. *A Brassica juncea* plant comprising an acetohydroxyacid synthase large subunit (AHASL) polynucleotide encoding a herbicide-tolerant AHASL polypeptide having a threonine substitution at a position corresponding to position 122 of SEQ ID NO: 1, position 107 of SEQ ID NO: 4, or position 104 of SEQ ID NO: 5, wherein the AHASL polynucleotide is non-transgenic, wherein said plant comprises
(a) both (1) an A genome comprising the AHASL polynucleotide encoding the AHASL polypeptide having a threonine substitution at the position corresponding to position 104 of SEQ ID NO: 5 and (2) a B genome comprising an AHASL polynucleotide encoding a herbicide-tolerant AHASL polypeptide having an asparagine substitution at a position corresponding to position 638 of SEQ ID NO: 2, wherein said plant is homozygous for both of said AHASL polynucleotides, or
(b) both (1) a B genome comprising the AHASL polynucleotide encoding the AHASL polypeptide having a threonine substitution at the position corresponding to position 107 of SEQ ID NO: 4 and (2) an A genome comprising an AHASL polynucleotide encoding a herbicide-tolerant AHASL polypeptide having a leucine substitution at a position corresponding to position 556 of SEQ ID NO: 6, wherein said plant is homozygous for both of said AHASL polynucleotides.

2. The*Brassica juncea* plant of claim 1, wherein said plant:
a) is a progeny of line J04E-0122, a representative sample of seed of the line having been deposited under ATCC Patent Deposit Number PTA-7944;
b) is a mutant, recombinant, or a genetically engineered derivative of line J04E-0122, or
c) is a progeny of at least one of the plants of a) or b).

3. The *Brassica juncea* plant of claim 1, wherein said plant:
a) is a progeny of line J04E-0130, a representative sample of seed of the line having been deposited under ATCC Patent Deposit Number PTA-7945;
b) is a mutant, recombinant, or a genetically engineered derivative of line J04E-0130, or
c) is a progeny of at least one of the plants of a) or b).

4. A plant cell of the *Brassica juncea* plant of any one of claims 1 to 3.

5. A plant part of the *Brassica juncea* plant of any one of claims 1 to 3.

6. A seed of the *Brassica juncea* plant of any one of claims 1 to 3, wherein the seed comprises the AHASL polynucleotide(s) of any one of claims 1 to 3.

7. The seed of claim 6, wherein said seed is treated with a seed treatment formulation.

8. The seed of claim 7, wherein the seed treatment formulation comprises an herbicide selected from imidazolinones, sulfonylureas, triazolopyrimidines, pyrimidinyloxybenzoates, and mixtures thereof.

9. A method for growing *a Brassica juncea* plant of any one of claims 1 to 3, comprising applying an effective amount of an AHAS-inhibiting herbicide to the plant and the vicinity of the plant.

10. The method of claim 9, wherein the AHAS-inhibiting herbicide comprises one or more of an imidazolinone herbicide, a sulfonylurea herbicide, a triazolopyrimidine herbicide, and apyrimidinyloxybenzoate herbicide.

## Patentansprüche

1. *Brassica juncea-*Pflanze, umfassend ein Acetohydroxysäuresynthase-große-Untereinheit(AHASL)-Polynukleotid, das ein herbizidtolerantes AHASL-Polypeptid codiert, das eine Threonin-Substitution an einer Position aufweist, die Position 122 von SEQ ID NO: 1, Position 107 von SEQ ID NO: 4 oder Position 104 von SEQ ID NO: 5 entspricht, wobei das AHASL-Polynukleotid nicht transgen ist, wobei die Pflanze umfasst
(a) sowohl (1) ein A-Genom, das das AHASL-Polynukleotid umfasst, das das AHASL-Polypeptid mit einer Threonin-Substitution an der Position, die Position 104 von SEQ ID NO: 5 entspricht, codiert, als auch (2) ein B-Genom, das ein AHASL-Polynukleotid umfasst, das ein herbizidtolerantes AHASL-Polypeptid mit einer Asparagin-Substitution an einer Position, die Position 638 von SEQ ID NO: 2 entspricht, codiert, wobei die Pflanze für die beiden AHASL-Polynukleotide homozygot ist, oder
(b) sowohl (1) ein B-Genom, das das AHASL-Polynukleotid umfasst, das das AHASL-Polypeptid mit einer Threonin-Substitution an der Position, die Position 107 von SEQ ID NO: 4 entspricht, codiert, als auch (2) ein A-Genom, das ein AHASL-Polynukleotid umfasst, das ein herbizidtolerantes AHASL-Polypeptid mit einer Leucin-Substitution an einer Position, die Position 556 von SEQ ID NO: 6 entspricht, codiert, wobei die Pflanze für die beiden AHASL-Polynukleotide homozygot ist.

2. *Brassica juncea*-Pflanze nach Anspruch 1, wobei die Pflanze:
(a) ein Nachkomme der Linie J04E-0122 ist, wobei eine repräsentative Probe von Samen der Linie unter der ATCC-Patenthinterlegungsnummer PTA-7944 hinterlegt ist;
(b) ein mutiertes, rekombinantes oder ein gentechnisch verändertes Derivat der Linie J04E-0122 ist oder
(c) ein Nachkomme mindestens einer der Pflanzen unter a) oder b) ist.

3. *Brassica juncea*-Pflanze nach Anspruch 1, wobei die Pflanze:
(a) ein Nachkomme der Linie J04E-0130 ist, wobei eine repräsentative Probe von Samen der Linie unter der ATCC-Patenthinterlegungsnummer PTA-7945 hinterlegt ist;
(b) ein mutiertes, rekombinantes oder ein gentechnisch verändertes Derivat der Linie J04E-0130 ist oder
(c) ein Nachkomme mindestens einer der Pflanzen unter a) oder b) ist.

4. Pflanzenzelle der *Brassica juncea*-Pflanze nach einem der Ansprüche 1 bis 3.

5. Pflanzenteil der *Brassica juncea*-Pflanze nach einem der Ansprüche 1 bis 3.

6. Samen der *Brassica juncea*-Pflanze nach einem der Ansprüche 1 bis 3, wobei der Samen das bzw. die AHASL-Polynukleotid(e) nach einem der Ansprüche 1 bis 3 umfasst.

7. Samen nach Anspruch 6, wobei der Samen mit einer Samenbehandlungsformulierung behandelt ist.

8. Samen nach Anspruch 7, wobei die Samenbehandlungsformulierung ein aus Imidazolinonen, Sulfonylharnstoffen, Triazolopyrimidinen, Pyrimidinyloxybenzoaten und Gemischen davon ausgewähltes Herbizid umfasst.

9. Verfahren zum Züchten einer *Brassica juncea*-Pflanze nach einem der Ansprüche 1 bis 3, umfassend das Aufbringen einer wirksamen Menge eines AHASinhibierenden Herbizids auf die Pflanze und die Umgebung der Pflanze.

10. Verfahren nach Anspruch 9, wobei das AHASinhibierende Herbizid eines oder mehrere aus einem Imidazolinon-Herbizid, einem Sulfonylharnstoff-Herbizid, einem Triazolopyrimidin-Herbizid und einem Pyrimidinyloxybenzoat-Herbizid umfasst.

## Revendications

1. Végétal de type *Brassica juncea* comprenant un polynucléotide à grande sous-unité acétohydroxyacide synthase (AHASL) codant pour un polypeptide AHASL tolérant aux herbicides possédant une substitution de thréonine au niveau d'une position correspondant à la position 122 de la SEQ ID NO: 1, la position 107 de la SEQ ID NO: 4, ou la position 104 de la SEQ ID NO: 5, le polynucléotide AHASL étant non transgénique, ledit végétal comprenant
(a) à la fois (1) un génome A comprenant le polynucléotide AHASL codant pour le polypeptide AHASL possédant une substitution de thréonine au niveau de la position correspondant à la position 104 de la SEQ ID NO: 5 et (2) un génome B comprenant un polynucléotide AHASL codant pour un polypeptide AHASL tolérant aux herbicides possédant une substitution d'asparagine au niveau d'une position correspondant à la position 638 de la SEQ ID NO: 2, ledit végétal étant homozygote pour les deux dits polynucléotides AHASL, ou
(b) à la fois (1) un génome B comprenant le polynucléotide AHASL codant pour le polypeptide AHASL possédant une substitution de thréonine au niveau de la position correspondant à la position 107 de la SEQ ID NO: 4 et (2) un génome A comprenant un polynucléotide AHASL codant pour un polypeptide AHASL tolérant aux herbicides possédant une substitution de leucine au niveau d'une position correspondant à la position 556 de la SEQ ID NO: 6, ledit végétal étant homozygote pour les deux dits polynucléotides AHASL.

2. Végétal de type *Brassica juncea* selon la revendication 1, ledit végétal :
a) étant un descendant de la lignée J04E-0122, un échantillon représentatif de graine de la lignée ayant été déposé sous le numéro de dépôt de brevet ATCC PTA-7944 ;
b) étant un dérivé mutant, un dérivé recombinant, ou un dérivé modifié génétiquement de la lignée J04E-0122, ou
c) étant un descendant d'au moins un des végétaux de a) ou b).

3. Végétal de type *Brassica juncea* selon la revendication 1, ledit végétal :
a) étant un descendant de la lignée J04E-0130, un échantillon représentatif de graine de la lignée ayant été déposé sous le numéro de dépôt de brevet ATCC PTA-7945 ;
b) étant un dérivé mutant, un dérivé recombinant, ou un dérivé modifié génétiquement de la lignée J04E-0130, ou
c) étant un descendant d'au moins un des végétaux de a) ou b).

4. Cellule végétale du végétal de type *Brassica juncea* selon l'une quelconque des revendications 1 à 3.

5. Partie de végétal du végétal de type *Brassica juncea* selon l'une quelconque des revendications 1 à 3.

6. Graine du végétal de type *Brassica juncea* selon l'une quelconque des revendications 1 à 3, la graine comprenant le ou les polynucléotides AHASL selon l'une quelconque des revendications 1 à 3.

7. Graine selon la revendication 6, ladite graine étant traitée avec une formulation de traitement de graine.

8. Graine selon la revendication 7, la formulation de traitement de graine comprenant un herbicide choisi parmi des imidazolinones, des sulfonylurées, des triazolopyrimidines, des pyrimidinyloxybenzoates, et des mélanges correspondants.

9. Procédé pour la croissance d'un végétal de type *Brassica juncea* selon l'une quelconque des revendications 1 à 3, comprenant l'application d'une quantité efficace d'un herbicide inhibant AHAS au végétal et à la proximité du végétal.

10. Procédé selon la revendication 9, l'herbicide inhibant AHAS comprenant l'un ou plusieurs parmi un herbicide de type imidazolinone, un herbicide de type sulfonylurée, un herbicide de type triazolopyrimidine, et un herbicide de type pyrimidinyloxybenzoate.
